(19) Europäisches Patentamt
European Patent Office
Office européen des brevets

(11) Veröffentlichungsnummer: **0 575 977 A2**

(12) **EUROPÄISCHE PATENTANMELDUNG**

(21) Anmeldenummer: **93110010.1**

(51) Int. Cl.[5]: **A23G 3/30**, A61K 9/00

(22) Anmeldetag: **23.06.93**

(30) Priorität: **25.06.92 DE 4220735**

(43) Veröffentlichungstag der Anmeldung: **29.12.93 Patentblatt 93/52**

(84) Benannte Vertragsstaaten: **AT BE CH DE DK ES FR GB GR IE IT LI LU MC NL PT SE**

(71) Anmelder: **MEDICE Chem.-Pharm. Fabrik Pütter GmbH & Co. KG
Kuhloweg 37-39
D-58638 Iserlohn(DE)**

(72) Erfinder: **Szejtli, Jozsef, Prof.
c/ Cyclolab Research & Dev.,
Postfach 17
HU-1525 Budapest(HU)**
Erfinder: **Pütter, Sigurd, Dr.
c/o Medice,
Kuhloweg 37-39
D-5860 Iserlohn(DE)**

(74) Vertreter: **Reinhard, Skuhra, Weise
Postfach 44 01 51
D-80750 München (DE)**

(54) **Kaugummizubereitungen.**

(57) Es werden Kaugummizubereitungen mit erhöhter und zeitlich verlängerter Freisetzung der Inhalts- und/oder Wirkstoffe, Verfahren zur Herstellung dieser Kaugummizubereitung sowie Verwendungen hierfür offenbart.

EP 0 575 977 A2

Die Erfindung betrifft neue Kaugummizubereitungen mit erhöhter und zeitlich verlängerter Freisetzung der Inhalts- und/oder Wirkstoffe, Verfahren zur Herstellung dieser Kaugummizubereitungen sowie Verwendungen hierfür.

Kaugummi ist als Träger für Wirkstoffe, die lokal wirken oder von der Mundschleimhaut absorbiert werden sollen, eine wichtige Alternative zu herkömmlichen oralen Zubereitungen. Als Wirkstoffe in diesen Kaugummizubereitungen, z.B. zur Behandlung von lokalen Erkrankungen im Mund-Rachenraum, können Arzneimittel zur Behandlung von Zahnerkrankungen, Desinfektionsmittel und Arzneimittel zur Behandlung von Erkrankungen im Rachenraum verwendet werden (vgl. BE 71 216 (1974)). Die Freisetzung der Wirkstoffe genügt jedoch nicht den medizinischen Anforderungen, da der hydrophobe Wirkstoff mit großer Affinität an die hydrophobe Gummigrundlage gebunden ist. In der EP 0 299 929 (1988) und der EP 0 299 924 (1988) werden anästhetisch wirkende Wirkstoffe mit einem Stoff, z.B. Zucker, vorgemischt, der während des Kauens leicht freigesetzt wird. Die Freisetzungsgeschwindigkeit ist jedoch im allgemeinen zu Beginn sehr groß und nimmt nach längerem Kauen stark ab. Um eine gleichmäßige Wirkstofffreisetzung zu erreichen, wird das Anästhetikum in einem mikroporösen Stoff, nämlich einem löslichen Zucker wie Maltodextrin, eingeschlossen.

Die DE 37 32 677 (1989) offenbart eine Kaugummizubereitung mit einer Schichtstruktur, die einen Wirkstoff an der Grenzschicht enthält, d.h. der Wirkstoff ist nicht Teil der Gummimasse, wodurch eine fast kontinuierliche Freisetzung des Wirkstoffs über einen langen Zeitraum erfolgt. In der CA 107:235200 werden Kaugummizubereitungen beschrieben, in welchen der Wirkstoff in einer vernetzten, mehrwertigen Kation-Alginat- oder Carrageenat-Matrix eingeschlossen ist.

Die auflösungs-regulierende Wirkung von Cyclodextrinen wird bereits in verschiedenen pharmazeutischen Zubereitungen verwendet. Aus der DE-OS 30 08 663 (1980) sind Cyclodextrin-enthaltende Kaugummizubereitungen zur Vermeidung schlechten Atems bekannt. Die JP 146 396 (14.6.88) beschreibt Kaugummis, die Duftstoffe und/oder medizinische Wirkstoffe in Cyclodextrin oder methyliertem Cyclodextrin eingeschlossen enthalten. Es wird hier beschrieben, daß die Duftstoffe und/oder medizinischen Wirkstoffe aus der Kaugummigrundlage beim Kauen stetig und gleichmäßig abgegeben werden. Die Verwendung von Cyclodextrinpolymeren wird durch diese Druckschrift nicht nahegelegt.

Es ist eine Aufgabe der Erfindung, neue Kaugummizubereitungen bereitzustellen, durch welche die Freisetzung der Inhalts- und/oder Wirkstoffe kontinuierlich über einen längeren Zeitraum und in erhöhter Menge erfolgt.

Diese Aufgabe wird erfindungsgemäß dadurch gelöst, daß die Inhalts- und/oder Wirkstoffe mit einem quellfähigen Kohlenhydratpolymer einen Einschlußkomplex bilden.

Bevorzugt ist das quellfähige Kohlenhydratpolymer einer Stärke und/oder ein Stärkederivat, wobei das Stärkederivat bevorzugt durch chemische/oder enzymatische Reaktionen gewonnen wird. Insbesondere bevorzugt ist das quellfähige Kohlenhydratpolymer ein Cyclodextrinpolymer und/oder ein Cyclodextrinpolymerderivat.

Cyclodextrine sind cyclische Oligosaccharide mit 6 ($\alpha$-Cyclodextrin), 7 ($\beta$-Cyclodextrin) oder 8 ($\gamma$-Cyclodextrin) Glucopyranoseeinheiten. Ihre Herstellung erfolgt durch enzymatischen Abbau von Stärke. Derartige Cyclodextrine sind in der Lage, Einschlußkomplexe zu bilden. Das hydrophobe Innere des Cyclodextrinrings (Wirt) stellt eine bevorzugte Umgebung für das hydrophobe Gastmolekül oder für den hydrophoben Teil des Gastmoleküls, z.B. einem Inhalts- oder Wirkstoff, dar.

Das Kohlenhydratpolymer ist eine wasser-unlösliche, gequollene Stärke oder ein Stärkederivat, z.B. ein Cyclodextrinpolymer. Die erfindungsgemäß einsetzbaren Cyclodextrinpolymere sind $\alpha$-, $\beta$- oder $\gamma$-Cyclodextrinpolymere, die mit einem bi- oder polyfunktionellen Reagens vernetzt sind. Die Vernetzung erfolgt z.B. durch Epoxide (vgl. US 4 274 985 (1981)). Erfindungsgemäß sind auch Polymere verwendbar, die mit ionischen Gruppen (Carboxyalkyl-, Sulphalkyl-, Alkylamino-, Dialkylaminogruppen) und/oder hydrophoben Gruppen (Alkyl-, Arylalkyl-, Acyl-, Alkylsulphonyl-, Desoxyaminoalkyl- oder Silylgruppen) modifiziert sind. Die Herstellung dieser Cyclodextrinpolymere erfolgt gemäß den nachfolgend beschriebenen Verfahren. Weiterhin wird vollinhaltlich auf die in der ungarischen Patentanmeldung 6654/89 (1989) beschriebenen Herstellungsverfahren Bezug genommen. Ein in der erfindinngsgemäßen Kauginmmizubereitung bevorzugt einsetzbares Cyclodextrin weist die folgende Formel auf:

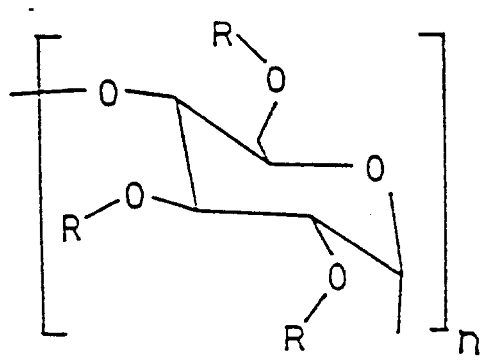

wobei n = 6 (α-Cyclodextrin), 7 (β-Cyclodextrin) oder 8 (γ-Cyclodextrin) und

R = H und/oder eine ionische Gruppe und/oder eine hydrophobe Gruppe und/oder eine vom Vernetzungsmittel stammende Gruppe ist, die zwei Cyclodextrinringe verbindet,

und/oder eine vom Vernetzungsmittel stammende und mit einem Cyclodextrin monofunktionell umgesetzte Gruppe ist,

und es in polymerer Form vorliegt.

Bevorzugt ist die ionische Gruppe eine Carboxyalkyl-, Sulphalkyl-, Alkylamino- und/oder Dialkylaminogruppe, wobei die Alkylgruppe bevorzugt ein $C_1$ - $C_5$-Alkylrest ist.

Bevorzugt ist die hydrophobe Gruppe eine Alkyl-, Arylalkyl-, Acyl-, Alkylsulphonyl-, Desoxyaminoalkyl- und/oder Silylgruppe, wobei der Alkylrest bevorzugt ein $C_1$ - $C_5$-Alkylrest ist.

Der Alkylrest ist bevorzugt ein verzweigter und/oder unverzweigter $C_1$ - $C_5$-Alkylrest, bevorzugt ein $C_1$ - $C_3$-Alkylrest.

Die vom Vernetzungsmittel stammende Gruppe, die zwei Cyclodextrinringe verbindet, ist bevorzugt eine Glyceryl-, Ethylenglykol-diglyceryl- und/oder Butylenglykoldiglyceryl-Gruppe.

Die vom Vernetzungsmittel stammende und mit einem Cyclodextrin monofunktionell umgesetzte Gruppe ist bevorzugt eine Dihydroxypropyl-, 2-Hydroxy-3-[2-(2,3-dihydroxy)propoxy]ethoxy)propyl-, (2-Hydroxy-3-[2-(2,3-dihydroxy)propoxy]butoxy)propylgruppe.

Das polymere Cyclodextrin enthält 30 - 70 %, bevorzugt 50 - 60 % an Cyclodextrin.

Das Cyclodextrin ist bevorzugt ein polymeres, dihydroxyalkyliertes Cyclodextrinderivat, welches weiterhin bevorzugt durch ionische Gruppen substituiert ist.

Der Substitutionsgrad am Cyclodextrinring liegt im Bereich von 0 - 21, bei Dimethyl-Cyclodextrin bevorzugt bei 14, Hydroxypropylcyclodextrin im Bereich von 1,2 - 8.

In Mischethern liegt der Substitutionsgrad der Alkylsubstituenten bevorzugt im Bereich von 0 - 14, wobei die Summe der Substituenten im Bereich von 0 - 21 liegt.

Weiterhin bevorzugt enthält die erfindungsgemäße Kaugummizubereitung die lipophilen Cyclodextrinpolymerderivate, die im Anschluß an die Ausführungsbeispiele 1 - 18 offenbart sind.

Das Kohlenhydratpolymer kann leicht zusammen mit den darin eingeschlossenen Inhalts- und/oder Wirkstoffen aus der hydrophoben Gummigrundlage ausgekaut werden. Das Kohlenhydratpolymer quillt im Speichel, wodurch sich eine verstärkte Wirkstofffreisetzung ergibt. Die Freisetzungsgeschwindigkeit des gequollenen Polymers hängt u.a. von der Stabilitätskonstante des Inhalts-/Wirkstoffs-Cyclodextrinkomplexes ab.

Die Komplexbildung kann durch ionische Wechselwirkungen, z.B. mit den ionischen Gruppen der Inhalts-/Wirkstoffe, gefördert werden. Erfindungsgemäß bevorzugt werden deshalb zur verzögerten Freisetzung basischer Inhalts-/Wirkstoffe kationische Polymere und zur verzögerten Freisetzung saurer Inhalts-/Wirkstoffe anionische Polymere verwendet. Die Gegenwart hydrophober Gruppen auf dem Polymer verstärkt die hydrophoben Wechselwirkungen, was für die Freisetzung der hydrophoben Wirkstoffe günstig ist. Die letztgenannten Polymerderivate binden jedoch mit einer gewissen Affinität an die Kaugummimasse, weshalb die Menge an insgesamt freigesetztem Inhalts-/Wirkstoff geringer ist als bei den hydrophilen Polieren. Erfindungsgemäß wird deshalb eine Kombination unterschiedlich substituierter Polymere bevorzugt, wodurch eine optimale Freisetzungskinetik erreicht werden kann.

Erfindungsgemäß können alle Wirkstoffe verwendet werden, die in der Lage sind, Einschlußkomplexe mit Cyclodextrinpolymeren einzugehen. Insbesondere bevorzugt sind Wirkstoffgruppen zur Behandlung von Erkrankungen des Mund-Rachenraums und des dentalen Bereichs sowie die Wirkstoffe, die oral absorbiert werden, beispielsweise Analgetika, Antiarhytmika, Antimykotika, antiinflammatorisch wirkende Mittel, Antipyretika, Antiinfektiva, Antihistaminika, psychotrop wirkende Mittel, Stimulantia, Sedativa, Tranquilizer, Laxativa,

gefäßerweiternde- und gefäßverengende Mittel, Diuretika, Antiasthmatika, Expectorantia, Mucolytika, Antibiotika, Antitumormittel, Antitussiva, Antihypertonika, Antihypotonika und Antithrombotika und Antikariesmittel, Antiallergika, Lokalanästhetika sowie deren Mischungen. Als desinfizierende Mittel verwendbar sind Stoffe wie Salicylsäure und ihre Derivate, Thymol, Menthol, Hexachlorophen, Chlorhexidin, Jod, Cetylpyridiniumsalze, Achridinderivate wie Xantachridin usw. oder lokal wirkende Anästhetika wie Etidocain, Tetracain, Chloroprocain, Dibucain, Bupivacain, Lidocain, Tripellamin, Benzocain, Hexylresorcinol usw.; als entzündungshemmende Wirkstoffe sind beispielsweise verwendbar Triamcinolonacetonid, Hydrocortison, Prednisolon, Indomethacin, Fluorbiprofen usw.. Als Antiallergika sind beispielsweise verwendbar Phenylpropanolamin, Chlorpheniramin, Dimethinden, Cyproheptadin, Phenindamin usw.. Als Antitussiva sind erfindungsgemäß verwendbar beispielsweise Dextromethorphan, Codein, Ephedrin usw.. Als Tranquilizer sind erfindungsgemäß verwendbar beispielsweise Chlorpromazin, Chlordiazepoxid usw.. Als Antikariesmittel sind erfindungsgemäß beispielsweise verwendbar Linolsäure, saure Triterpene, halogenierte Diphenylether, Benzolsäureester usw.. Als Laxativa sind erfindungsgemäß beispielsweise verwendbar Phenolphthalein, Sennozid, Bisacodyl usw.. Weiterhin sind erfindungsgemäß verwendbar Vitamine oder Aminosäuren und deren Derivate.

Als Inhaltsstoffe sind erfindungsgemäß einsetzbar natürliche und/oder naturidentische Aromastoffe, die mit den quellfähigen Kohlenhydratpolymeren, insbesondere den Cyclodextrinpolymeren, Komplexe bilden. Beispiele hierfür sind Pfefferminzöl, Menthol, Vanillin, Zimt, Anis, Fruchtaromen wie Himbeere, Erdbeere, Zitrone, Orange, Aprikose, Pfirsich, Kiwi, Mango, Banane und Fruchtmischungen und/oder künstliche Süßstoffe wie beispielsweise Aspartam.

Erfindungsgemäß besteht die Kaugummizusammensetzung aus den herkömmlich verwendeten Kaugummigrundlagen, ergänzt durch herkömmliche Kaugummibestandteile wie Weichmacher, Füllstoffe, Geschmacks- und/oder Süßstoffe. Als Kaugummigrundmasse können alle wasser-unlöslichen, natürlichen oder synthetischen Gummigrundlagen, im allgemeinen im Anteil von 5 - 50 Gew.-%, bevorzugt 15 - 30 Gew.-% , der Kaugummizusammensetzung verwendet werden.

Herkömmliche Additive sind : Emulgatoren wie Glycerin und Glycerylmonostearat; geschmacksbildende Zusätze natürlichen oder synthetischen Ursprungs wie Grüne Minze, Zimt, Pfefferminze; Farbstoffe wie Titandioxyd oder andere, lebensmittel- oder arzneimittelrechtlich zugelassene Farbstoffe.

Zur Süßung können der Kaugummizubereitung natürliche oder künstliche Süßstoffe zugegeben werden. Beispiele hierfür sind Saccharose, Glucose, Dextrose, Sorbitol, Xylitol, Mannitol, Saccharin, Cyclamat oder Aspartam. Die Menge der natürlichen Süßstoffe beträgt im allgemeinen 20 - 60 Gew.-%, bevorzugt 40 - 60 Gew.-% der Kaugummizubereitung. Bei den künstlichen Süßstoffen beträgt die zugegebene Menge im allgemeinen 0,001 - 5 Gew.-%, bevorzugt etwa 0,05 - 1 Gew.-% der Kaugummizubereitung.

Der oder die Wirkstoffe sind in Mengen von 0,001 - 1 Gew.-%, das Kohlenhydratpolymer in Mengen von 1- 50 Gew.-%, bevorzugt von 2 - 10 Gew.-%, je nach Menge des Wirkstoffs, in der Kaugummizubereitung vorhanden. Wenn als Kohlenhydratpolymer ein Cyclodextrinpolymer verwendet wird, liegt das molare Verhältnis von Wirkstoff : Cyclodextrin im Bereich von 1:5 - 3:1 . Bevorzugt ist ein molares Verhältnis von 1:1. Die Teilchengröße des Polymers beträgt weniger als 100 µm, bevorzugt weniger als 60 µm und mehr als 10 µm, um die organoleptischen Eigenschaften des Gummis nicht zu vermindern.

Die Herstellung der erfindungsgemäßen Zubereitungen aus Polymer und Wirkstoff erfolgt durch

a) quellen des Polymers in einer geeigneten Lösung des Inhalts-/Wirkstoffs oder

b) durch Mischen des Polymers und des Inhalts-/Wirkstoffs im trocknen Zustand und anschließendes Quellen in einer geeigneten Lösung.

Die Suspension wird bevorzugt gerührt oder geschüttelt, falls notwendig anschließend gefiltert und getrocknet. Zur Auflösung des Inhalts-/Wirkstoffs und zum Quellen kann eine wäßrige, wäßrig-alkoholische oder eine Pufferlösung verwendet werden. Das Endprodukt kann bei Zimmertemperatur oder bei erhöhten Temperaturen getrocknet werden, je nach den Eigenschaften der verwendeten Inhalts-/Wirkstoffe. Die Quell- und Rührschritte werden bei 0 - 60 °C , bevorzugt bei Zimmertemperatur, für 0,5 - 24 Stunden, bevorzugt bei 1 - 6 Stunden, durchgeführt. Wenn eine Mischung verschiedener Inhalts-/Wirkstoffe verwendet wird, kann zur Bildung des Polymers die zur Auflösung des Inhalts-/Wirkstoffs verwendete Lösung verwendet werden oder die Polymer-Inhalts-/Wirkstoff-Zubereitungen können entweder in gequollenem oder trocknem Zustand vermischt werden.

Die Kaugummizubereitungen werden durch herkömmliche Verfahren hergestellt. In einem bevorzugten Verfahren wird die Kaugummigrundlage auf 50 - 120 °C in einem Knetgerät erhitzt und anschließend die Weichmacher, Emulgatoren, Geschmacks- und Farbmittel zugegeben, worauf die Inhaltsstoff-/Wirkstoff-Kohlenhydratpolymerzubereitung und das Süßmittel gemeinsam oder getrennt zugefügt werden. Die Mischung wird nach jeder Zugabe so lange geknetet, bis eine homogene Masse erreicht wird. Im Anschluß wird die Kaugummizubereitung in eine geeignete Form gebracht.

Die erfindungsgemäß bereitgestellten Kaugummizubereitungen zeigen eine kontinuierliche Freisetzung der Inhalts- und/oder Wirkstoffe über einen längeren Zeitraum im Vergleich zu den aus dem Stand der Technik bekannten Kaugummizubereitungen. Weiterhin werden die Inhalts-/Wirkstoffe in größerer Menge freigesetzt als bei herkömmlichen Kaugummizubereitungen. In Folge der Komplexbildung mit dem Kohlenhydratpolymer, insbesondere dem polymeren Cyclodextrin, ist der unangenehme Geschmack mancher Wirkstoffe vermindert.

Beispiel 1

Kaugummizubereitungen mit einem Gehalt von 5 mg Cetylpyridiniumchlorid/g in Form des freien Wirkstoffs oder komplexiert mit einem Cyclodextrinpolymer ($\beta$-Cyclodextringehalt im Polymer: 56 Gew.-%, Cetylpyridiniumchloridgehalt im Komplex 12,5 Gew.-%) werden wie folgt hergestellt :

| | 1-1 | 1-2 |
|---|---|---|
| Gummigrundlage | 24,0 | 24,0 |
| Getreidesirup | 15,0 | 15,0 |
| Glycerin | 2,0 | 2,0 |
| Sojalecithin | 1,0 | 1,0 |
| Cetylpyridiniumchlorid (CPC) | 0,5 | - |
| Cetylpyridiniumchloridcyclodextrinpolymerkomplex (CPC-CDP) | - | 4,0 |
| pulverisierter Zucker | 57,5 | 54,0 |

Die Gummizusammensetzungen werden hergestellt durch das Erwärmen der Gummigrundmasse in einem Kneter auf etwa 70 °C, anschließende Zugabe von Glycerin, Sojalecithin und Stärkesirup, sowie Zugabe des Wirkstoffes und des Zuckers. Die Zusammensetzung wird nach jeder Zugabe bis zum Erreichen einer homogenen Masse geknetet.

Die Kaugummizubereitungen wurden einzelnen Testversuchen unterzogen. Die Menge des während des Kauens freigesetzten Cetylpyridiniumchlorids wurde durch das Messen des Wirkstoffgehalts in der Kaugummikugel nach dem Kauen nach einer geeigneten Zeit ( 5 und 30 Min.) bestimmt.

Die Kaugummizubereitung ist gekennzeichnet durch die Menge an freigesetztem Cetylpyridiniumchlorid nach 30 Minuten kauen und durch den Retardierungskfaktor, der sich bestimmt durch die Menge des nach fünfminütigem Kauen freigesetzten Cetylpyridiniumchlorids im Verhältnis zum freigesetzten Cetylpyridiniumchlorid nach 30 Minuten Kauen.

| No. | Wirkstoff | Cetylpyridiniumchloridfreisetzung in 30 Min. (%) | Retardierungsfaktor (%) |
|---|---|---|---|
| 1-1 | CPC | 26 | 96 |
| 1-2 | CPC-CDP | 48 | 89 |
| Durchschnittswerte nach 5 Parallelexperimenten (einschl. der Gummiherstellung) | | | |

Die Ergebnisse zeigen, daß aus den erfindungsgemäßen Kaugummizubereitungen ein signifikant größerer Prozentsatz an CPC freigesetzt wurde als aus den Kontrollzusammensetzungen. Weiterhin ist eine leichte Verbesserung im Retardierungsfaktor beobachtbar.

Beispiel 2

Gemäß Beispiel 1 hergestellte Kaugummizubereitungen unter Verwendung von Carboxymethylstärke (CMA) und Carboxymethylcyclodextrinpolymer (CM-CDP) mit unterschiedlichem Substitutionsgrad anstelle eines Cyclodextrinpolymeren zur Komplexbildung.

| No. | Wirkstoff | Cetylpyridiniumchloridfreisetzung in 30 Min. (%) | Retardierungsfaktor (%) |
|---|---|---|---|
| 2-1 | CMA (COOH:0,3%) | 44 | 95 |
| 2-2 | CPC-CM-CDP (COOH:0,6%) | 54 | 75 |
| 2-3 | CPC-CM-CDP (COOH:2,0%) | 55 | 62 |
| 2-4 | CPC-CM-CDP-löslich (COOH:4,0%) | 49 | 95 |
| Durchschnittswerte aus 2 Parallelexperimenten (einschl. der Gummiherstellung) | | | |

Die Ergebnisse zeigen, daß die Menge des in 30 Min. freigesetzten Cetylpyridiniumchlorids praktisch unabhängig vom Carboxylgehalt des Cyclodextrinpolymeren ist, jedoch der Retardierungsfaktor mit zunehmendem Carboxylgehalt im Falle der nichtlöslichen, quellfähigen Polymere signifikant verbessert wird. Das lösliche Polymer hat, obwohl es den höchsten Carboxylgehalt aufweist, keinen Einfluß auf den Retardierungsfaktor.

Beispiel 3

Gemäß Beispiel 1 und Beispiel 2 werden Kaugummizubereitungen unter Verwendung des in der Zubereitung No. 1-2 verwendeten Komplexes hergestellt, modifiziert mit 1% Aerosil und 3% Honigwachs; Der in der Zubereitung No. 2-3 verwendete Komplex wird mit 5% Aerosil als hydrophobem Hilfsgast modifiziert.

| No. | Wirkstoff | Cetylpyridiniumchloridfreisetzung in 30 Min. (%) | Retardierungsfaktor (%) |
|---|---|---|---|
| 3-1 | CPC-CDP + 1% Aerosil | 60 | 75 |
| 3-2 | CPC-CM-CDP (COOH:2,0%) + 5% Aerosil | 59 | 58 |
| 3-3 | CPC-CDP + 3% Honigwachs | 57 | 74 |
| Durchschnittswerte aus 2 Parallelexperimenten (einschl. der Gummiherstellung) | | | |

Die Modifikation mit Aerosil oder Honigwachs verbessert sowohl die Menge an freigesetztem Cetylpyridiniumchlorid als auch den Retardierungsfaktor.

Beispiel 4

Kaugummizubereitungen, enthaltend 3 mg Lidocain/g in Form des freien Wirkstoffs oder komplexiert mit einem Cyclodextrinpolymeren ($\beta$-Cyclodextringehalt im Polymer: 56 %, Lidocaingehalt im Komplex 10,5 %) werden wie folgt hergestellt :

| | 4-1 | 4-2 | 4-3 | 4-4 |
|---|---|---|---|---|
| Gummigrundmasse | 30,0 | 30,0 | 30,0 | 30,0 |
| 70% Sorbitollösung | 23,0 | 23,0 | 23,0 | 23,0 |
| Glycerin | 2,0 | 2,0 | 2,0 | 2,0 |
| Lidocain | 0,3 | - | - | - |
| Lidocain-CDP-Komplex | - | 2,9 | - | - |
| Lidocain-CM-CDP (COOH:2,0%) | - | - | 2,9 | - |
| Lidocain-Butyl-CDP | - | - | - | 2,9 |
| Pfefferminzöl | 0,5 | 0,5 | 0,5 | 0,5 |
| Sorbitolpulver | 44,2 | 41,6 | 41,6 | 41,6 |
| Die Gummizusammensetzungen werden wie in Beispiel 1 angegeben hergestellt. | | | | |

Alle erfindungsgemäßen Zubereitungen zeigen eine höhere Wirkstofffreisetzung im Vergleich zu den herkömmlichen Zubereitungen (4-1). Beim Kauen der Zubereitungen No. 4-3 und 4-4 wird eine gleichmäßige Lidocainfreisetzung erzielt.

Beispiel 5

Kaugummizubereitungen, enthaltend 1,5 mg Hydrocortison/g in Form des freien Wirkstoffs oder komplexiert mit einem Cyclo-dextrinpolymer ($\beta$-Cyclodextrin-Gehalt im Polymer : 56 %, Hydrocortisongehalt im Komplex 8,0 %) werden wie folgt hergestellt:

| | 5-1 | 5-2 | 5-3 |
|---|---|---|---|
| Gummigrundmasse | 37,0 | 37,0 | 37,0 |
| 60% Xylitollösung | 15,0 | 15,0 | 15,0 |
| Emulgator | 3,0 | 3,0 | 3,0 |
| Hydrocortison | 0,15 | - | - |
| Hydrocortison-CDP-Komplex | - | 1,9 | - |
| Hydrocortison-Methyl-CDP | - | - | 1,9 |
| Zitronenöl | 0,9 | 0,9 | 0,9 |
| Sorbitolpulver | 44,0 | 42,2 | 42,2 |
| Die Gummizubereitungen werden wie im Beispiel 1 angegeben hergestellt . | | | |

Alle erfindungsgemäßen Zubereitungen zeigen eine höhere Wirkstofffreisetzung im Vergleich zu den herkömmlichen Formulierungen (5-1). Beim Kauen der Zubereitung No. 5-3 wird eine gleichmäßige Lidocain-Freisetzung erzielt.

Beispiel 6

Kaugummizubereitungen, enthaltend 2 mg Chlorpheniramin/g in Form des freien Wirkstoffs oder komplexiert mit einem Cyclodextrinpolymer ($\beta$-Cyclodextrin-Gehalt im Polymer : 56 %, Chlorpheniramin-Gehalt im Komplex 10 %) werden wie folgt hergestellt:

| | 6-1 | 6-2 | 6-3 | 6-4 |
|---|---|---|---|---|
| Gummigrundmasse | 22,0 | 22,0 | 22,0 | 22,0 |
| Getreidesirup | 14,0 | 14,0 | 14,0 | 14,0 |
| Glycerin | 0,5 | 0,5 | 0,5 | 0,5 |
| Lecithin | 0,2 | 0,2 | 0,2 | 0,2 |
| Farbe | 0,1 | 0,1 | 0,1 | 0,1 |
| Chlorpheniramin | 0,2 | - | - | - |
| Chlorpheniramin-CDP-Komplex | - | 2,0 | - | - |
| Chlorpheniramin-CM-CDP (C00H:2,0 %) | - | - | 2,0 | - |
| Chlorpheniramin-Silyl-CDP | - | - | - | 2,0 |
| Menthol | 0,5 | 0,5 | 0,5 | 0,5 |
| pulverförmiger Zucker | 62,5 | 60,5 | 60,5 | 60,5 |
| Die Gummizubereitungen werden wie im Beispiel 1 angegeben hergestellt . | | | | |

Alle erfindinngsgemäßen Zubereitungen zeigen eine höhere Wirkstofffreisetzung im Vergleich zu den herkömmlichen Formulierungen (6-1). Beim Kauen der Zubereitungen No. 6-3 und 6-4 wird eine gleichmäßige Lidocain-Freisetzung erzielt.

Beispiel 7

Kaugummizubereitungen, enthaltend 7 mg Vitamin $B_1$ / g in Form des freien Wirkstoffs oder komplexiert mit einem Cyclodextrinpolymer ($\beta$-Cyclodextrin-Gehalt im Polymer : 56 %, Vitamin $B_1$ Gehalt im Komplex 15 %) werden wie folgt hergestellt:

| | 7-1 | 7-2 | 7-3 | 7-4 |
|---|---|---|---|---|
| Gummigrundmasse | 33,0 | 33,0 | 33,0 | 33,0 |
| 60% Sorbitollösung | 20,0 | 20,0 | 20,0 | 20,0 |
| Glycerin | 1,3 | 1,3 | 1,3 | 1,3 |
| Sojalecithin | 0,6 | 0,6 | 0,6 | 0,6 |
| Farbe | 0,1 | 0,1 | 0,1 | 0,1 |
| Vitamin $B_1$ | 0,7 | - | - | - |
| Vitamin $B_1$-CDP-Komplex | - | 4,7 | - | - |
| Vitamin $B_1$-Sulphopropyl-CDP | - | - | 4,7 | - |
| Vitamin $B_1$-Acetyl-CDP | - | - | - | 4,7 |
| Pfefferminzöl | 0,5 | 0,5 | 0,5 | 0,5 |
| Sorbitolpulver | 43,8 | 39,8 | 39,8 | 39,8 |
| Die Gummizubereitungen werden wie im Beispiel 1 angegeben hergestellt . | | | | |

Alle erfindinngsgemäßen Zubereitungen zeigen eine höhere Wirkstofffreisetzung im Vergleich zu den herkömmlichen Formulierungen (7-1). Beim Kauen der Zubereitungen No. 7-3 und 7-4 wird eine gleichmäßige Lidocain-Freisetzung erzielt.

Beispiel 8

Kaugummizubereitungen mit verzögerter Freisetzung der Geschmacks- und/oder Süßstoffe, enthaltend Zitronenöl und Aspartam in freier Form oder komplexiert mit einem Cyclodextrinpolymer ($\beta$-Cyclodextringehalt im Polymer : 63 %, Zitronenölgehalt im Komplex 10,0 %, Aspartamgehalt im Komplex 7,4 %) werden wie folgt hergestellt :

| | 8-1 | 8-2 | 8-3 |
|---|---|---|---|
| Gummigrundmasse | 25,0 | 25,0 | 25,0 |
| Mannitol | 18,0 | 18,0 | 18,0 |
| Emulgator | 5,0 | 5,0 | 5,0 |
| Zitronenöl | 1,0 | - | - |
| Zitronenöl-CDP-Komplex | - | 10,0 | - |
| Zitronenöl-Methyl-CDP | - | - | 10,0 |
| Aspartam | 0,3 | - | - |
| Aspartam-CDP-Komplex | - | 4,0 | 4,0 |
| Sorbitol Pulver | 50,7 | 38,0 | 38,0 |
| Die Gummizubereitungen werden wie im Beispiel 1 angegeben hergestellt . | | | |

Die erfindungsgemäßen Zubereitungen 8-2 und 8-3 zeigen eine erhöhte und verzögerte Freisetzung der Inhaltsstoffe im Vergleich zur herkömmlichen Formulierung 8-1.

Beispiel 9

Kaugummizubereitungen, enthaltend 20 mg Chlorpromazin/g in Form des freien Wirkstoffs oder komplexiert mit einem Cyclodextrinpolymer ($\beta$-Cyclodextringehalt im Polymer: 56 %, Chlorpromazingehalt im Komplex 12 %), werden wie folgt hergestellt:

| | 9-1 | 9-2 | 9-3 | 9-4 |
|---|---|---|---|---|
| Gummigrundmasse | 30,0 | 30,0 | 30,0 | 30,0 |
| 70% Sorbitollösung | 23,0 | 23,0 | 23,0 | 23,0 |
| Glycerin | 2,0 | 2,0 | 2,0 | 2,0 |
| Chlorpromazin | 2,0 | - | - | - |
| Chlorpromazin-$\beta$CDP-Komplex | - | 16,6 | - | - |
| Chlorpromazin-CM-$\beta$CDP (COOH: 2%) | - | - | 16,6 | - |
| Chlorpromazin-Butyl-$\beta$CDP | - | - | - | 16,6 |
| Pfefferminzöl | 0,5 | 0,5 | 0,5 | 0,5 |
| Sorbitolpulver | 42,5 | 27,9 | 27,9 | 27,9 |
| Die Gummizubereitungen werden wie in Beispiel 1 angegeben hergestellt. | | | | |

Alle erfindungsgemäßen Zubereitungen zeigten eine höhere Wirkstofffreisetzung im Vergleich zu den herkömmlichen Formulierungen (9-1). Eine verzögerte Freisetzung von Chlorpromazin wird beim Kauen der Formulierungen 9-3 und 9-4 erreicht.

Beispiel 10

Kaugummizusammensetzungen, enthaltend 10 mg Linolsäure/g in Form des freien Wirkstoffs oder komplexiert mit einem Cyclodextrinpolymer ($\beta$-Cyclodextringehalt im Polymer: 56 %; Linolsäuregehalt im Komplex 7,6 %), wurden wie folgt hergestellt:

| | 10-1 | 10-2 | 10-3 | 10-4 |
|---|---|---|---|---|
| Gummigrundmasse | 33,0 | 33,0 | 33,0 | 33,0 |
| 70% Sorbitollösung | 20,0 | 20,0 | 20,0 | 20,0 |
| Glycerin | 1,3 | 1,3 | 1,3 | 1,3 |
| Sojalecithin | 0,6 | 0,6 | 0,6 | 0,6 |
| Farbstoff | 0,1 | 0,1 | 0,1 | 0,1 |
| Linolsäure | 1,0 | - | - | - |
| Linolsäure-$\beta$CDP-Komplex | - | 13,1 | - | - |
| Linolsäure-Aminoethyl-$\beta$CDP | - | - | 13,1 | - |
| Linolsäure-Acetyl-$\beta$CDP | - | - | - | 13,1 |
| Pfefferminzöl | 0,5 | 0,5 | 0,5 | 0,5 |
| Sorbitolpulver | 43,5 | 31,4 | 31,4 | 31,4 |
| Die Gummizubereitungen werden wie in Beispiel 1 angegeben hergestellt. | | | | |

Alle erfindinngsgemäßen Zubereitungen zeigen eine erhöhte Freisetzung der Wirkstoffe im Vergleich zu den herkömmlichen Formulierungen (10-1). In den Formulierungen 10-3 und 10-4 wird beim Kauen eine verzögerte Freisetzung der Linolsäure erreicht.

Beispiel 11

Kaugummizubereitungen, enthaltend 100 mg Phenolphthalein/g in Form des freien Wirkstoffs oder komplexiert mit einem Cyclodextrinpolymer ($\beta$-Cyclodextringehalt im Polymer: 56 %, Phenolphthaleingehalt im Komplex 28 %), werden wie folgt hergestellt:

| | 11-1 | 11-2 |
|---|---|---|
| Gummigrundmasse | 24,0 | 24,0 |
| Getreidesirup | 15,0 | 15,0 |
| Glycerin | 2,0 | 2,0 |
| Sojalecithin | 1,0 | 1,0 |
| Phenolphthalein | 10,0 | - |
| Phenolphthalein-β-Cyclodextrinpolymerkomplex | - | 35,7 |
| Zucker, pulverförmig | 48,0 | 22,3 |
| Die Gummizubereitungen werden wie im Beispiel 1 angegeben hergestellt. | | |

Die erfindungsgemäße Formulierung 11-2 zeigt eine höhere Freisetzung des Wirkstoffs im Vergleich zur herkömmlichen Formulierung 11-1.

Beispiel 12

Kaugummizubereitungen, enthaltend 3 mg Dextrometorphan/g in Form des freien Wirkstoffs oder komplexiert mit einem Cyclodextrinpolymer (β-Cyclodextringehalt im Polymer: 56 %; Dextrometorphangehalt im Komplex 3,1 %), werden wie folgt hergestellt:

| | 12-1 | 12-2 |
|---|---|---|
| Gummigrundmasse | 24,0 | 24,0 |
| Getreidesirup | 15,0 | 15,0 |
| Glycerin | 2,0 | 2,0 |
| Sojalecithin | 1,0 | 1,0 |
| Dextrometorphan | 0,5 | - |
| Dextrometorphan-β-Cyclodextrinpolymerkomplex | - | 16,1 |
| Zucker, pulverförmig | 57,5 | 41,9 |
| Die Gummizubereitungen werden wie im Beispiel 1 angegeben hergestellt. | | |

Die erfindungsgemäße Zubereitung 12-2 zeigt eine höhere Wirkstofffreisetzung im Vergleich zur herkömmlichen Formulierung 12-1.

Beispiel 13

Kaugummizubereitungen, enthaltend 4 mg Menthol/g in Form des freien Wirkstoffs oder komplexiert mit einem Cyclodextrinpolymer (α-Cyclodextringehalt im Polymer: 47 %; Mentholgehalt im Komplex 7,4 %), werden wie folgt hergestellt:

| | 13-1 | 13-2 | 13-3 |
|---|---|---|---|
| Gummigrundmasse | 37,0 | 37,0 | 37,0 |
| 60% Xylitol-Lösung | 15,0 | 15,0 | 15,0 |
| Emulgator | 3,0 | 3,0 | 3,0 |
| Menthol | 0,4 | - | - |
| Menthol-αCDP-Komplex | - | 5,4 | - |
| Menthol-Ethyl-αCDP | - | - | 5,4 |
| Zitronenöl | 0,9 | 0,9 | 0,9 |
| Sorbitolpulver | 43,7 | 38,7 | 38,7 |
| Die Gummizubereitungen werden wie im Beispiel 1 angegeben hergestellt. | | | |

Alle erfindinngsgemäßen Zubereitungen zeigen eine höhere Freisetzung des Wirkstoffs im Vergleich mit der herkömmlichen Formulierung 13-1. Beim Kauen der Zubereitung 13-3 wird eine verzögerte Freisetzung des Menthols erreicht.

Beispiel 14

Kaugummizubereitungen, enthaltend 2 mg Triamcinolonacetonid/g in Form des freien Wirkstoffs oder komplexiert mit einem γ-Cyclodextrinpolymer (γ-Cyclodextringehalt im Polymer: 51 %; Triamcinolonacetonidgehalt im Komplex 14 %), werden wie folgt hergestellt:

| | 14-1 | 14-2 | 14-3 |
|---|---|---|---|
| Gummigrundmasse | 37,0 | 37,0 | 37,0 |
| 60% Xylitol-Lösung | 15,0 | 15,0 | 15,0 |
| Emulgator | 3,0 | 3,0 | 3,0 |
| Triamcinolonacetonid | 0,2 | - | - |
| Triamcinolonacetonid-γCDP-Komplex | - | 1,4 | - |
| Triamcinolonacetonid-Butyl-γCDP | - | - | 1,4 |
| Zitronenöl | 0,9 | 0,9 | 0,9 |
| Sorbitolpulver | 43,9 | 42,7 | 42,7 |
| Die Gummizubereitungen werden wie im Beispiel 1 angegeben hergestellt. | | | |

Alle erfindinngsgemäßen Zubereitungen zeigen eine höhere Freisetzung des Wirkstoffs im Vergleich mit der herkömmlichen Formulierung 14-1. Beim Kauen der Zubereitung 14-3 wird eine verzögerte Freisetzung des Triamcinolonacetonids erreicht.

Beispiel 15

Kaugummizubereitungen, enthaltend 3 mg Tetracain/g in Form des freien Wirkstoffs oder komplexiert mit einem Cyclodextrinpolymer (β-Cyclodextringehalt im Polymer: 56 %, Tetracaingehalt im Komplex 4,4 %), werden wie folgt hergestellt:

| | 15-1 | 15-2 | 15-3 | 15-4 |
|---|---|---|---|---|
| Gummigrundmasse | 30,0 | 30,0 | 30,0 | 30,0 |
| 70% Sorbitollösung | 23,0 | 23,0 | 23,0 | 23,0 |
| Glycerin | 2,0 | 2,0 | 2,0 | 2,0 |
| Tetracain | 0,3 | - | - | - |
| Tetracain-βCDP-Komplex | - | 6,8 | - | - |
| Tetracain-CM-βCDP (COOH:2%) | - | - | 6,8 | - |
| Tetracain-Silyl-βCDP | - | - | - | 6,8 |
| Pfefferminzöl | 0,5 | 0,5 | 0,5 | 0,5 |
| Sorbitolpulver | 44,2 | 37,7 | 37,7 | 37,7 |
| Die Gummizubereitungen werden wie in Beispiel 1 angegeben hergestellt. | | | | |

Alle erfindinngsgemäßen Zubereitungen zeigen eine höhere Freisetzung des Wirkstoffs im Vergleich mit der herkömmlichen Formulierung 15-1. Beim Kauen der Zubereitung 15-3 wird eine verzögerte Freisetzung des Tetracains erreicht.

Beispiel 16

Kaugummizubereitungen, enthaltend 25 mg Phenylbutazon und 1 mg Prednisolon in 1 g des Gummis werden hergestellt. Prednisolon wird in Form des freien Wirkstoffs oder komplexiert mit einem Cyclodextrinpolmer (/β-Cyclodextringehalt im Polymer: 56 %; Prednisolongehalt im Komplex 7,7 %) eingesetzt. Die Zubereitungen werden wie folgt hergestellt:

| | 16-1 | 16-2 | 16-3 |
|---|---|---|---|
| Gummigrundmasse | 37,0 | 37,0 | 37,0 |
| 60% Xylitol-Lösung | 15,0 | 15,0 | 15,0 |
| Emulgator | 3,0 | 3,0 | 3,0 |
| Phenylbutazon | 2,5 | 2,5 | 2,5 |
| Prednisolon | 0,1 | - | - |
| Prednisolon-$\beta$CDP-Komplex | - | 1,3 | - |
| Prednisolon-Methyl-$\beta$CDP | - | - | 1,3 |
| Zitronenöl | 0,9 | 0,9 | 0,9 |
| Sorbitolpulver | 41,5 | 40,3 | 40,3 |
| Die Gummizubereitungen werden wie im Beispiel 1 angegeben hergestellt. | | | |

Alle erfindinngsgemäßen Zubereitungen zeigen eine höhere Freisetzung des Wirkstoffs im Vergleich mit der herkömmlichen Formulierung 16-1. Beim Kauen der Zubereitung 16-3 wird eine verzögerte Freisetzung des Prednisolons erreicht.

Beispiel 17

Kaugummizubereitungen, enthaltend 8 mg Codein/g in Form des freien Wirkstoffs oder komplexiert mit einem Cyclodextrinpolymer ($\beta$-Cyclodextringehalt im Polymer: 56 %; Codeingehalt im Komplex 6,2 %), werden wie folgt hergestellt:

| | 17-1 | 17-2 | 17-3 |
|---|---|---|---|
| Gummigrundmasse | 37,0 | 37,0 | 37,0 |
| 60% Xylitol-Lösung | 15,0 | 15,0 | 15,0 |
| Emulgator | 3,0 | 3,0 | 3,0 |
| Codein | 0,8 | - | - |
| Codein-CDP-Komplex | - | 12,9 | - |
| Codein-Methyl-CDP | - | - | 12,9 |
| Zitronenöl | 0,9 | 0,9 | 0,9 |
| Sorbitolpulver | 43,3 | 31,2 | 31,2 |
| Die Gummizubereitungen werden wie im Beispiel 1 angegeben hergestellt. | | | |

Alle erfindinngsgemäßen Zubereitungen zeigen eine höhere Freisetzung des Wirkstoffs im Vergleich mit der herkömmlichen Formulierung 17-1. Beim Kauen der Zubereitung 17-3 wird eine verzögerte Freisetzung des Codeins erreicht.

Beispiel 18

Kaugummizubereitungen, enthaltend 5 mg Chlordiepoxid/g in Form des freien Wirkstoffs oder komplexiert mit einem Cyclodextrinpolymer ($\beta$-Cyclodextringehalt im Polymer: 56 %; Chlordiepoxidgehalt im Komplex 8,1 %), werden wie folgt hergestellt:

| | 18-1 | 18-2 | 18-3 | 18-4 |
|---|---|---|---|---|
| Gummigrundmasse | 30,0 | 30,0 | 30,0 | 30,0 |
| 70% Sorbitollösung | 23,0 | 23,0 | 23,0 | 23,0 |
| Glycerin | 2,0 | 2,0 | 2,0 | 2,0 |
| Chlordiepoxid | 0,5 | - | - | - |
| Chlordiepoxid-CDP-Komplex | - | 6,2 | - | - |
| Chlordiepoxid-CM-βCDP (COOH: 2,0%) | - | - | 6,2 | - |
| Chlordiepoxid-Butyl-CDP | - | - | - | 6,2 |
| Pfefferminzöl | 0,5 | 0,5 | 0,5 | 0,5 |
| Sorbitolpulver | 44,0 | 38,3 | 38,3 | 38,3 |
| Die Gummizubereitungen werden wie in Beispiel 1 angegeben hergestellt. | | | | |

Alle erfindinngsgemäßen Zubereitungen zeigen eine höhere Freisetzung des Wirkstoffs im Vergleich mit der herkömmlichen Formulierung 18-1. Beim Kauen der Zubereitung 18-3 wird eine verzögerte Freisetzung des Chlordiepoxids erreicht.

Im folgenden werden weitere neue lipophile Cyclodextrinpolymerderivate sowie Verfahren zur Herstellung dieser Cyclodextrinpolymerderivate offenbart, die in den erfindungsgemäßen Kaugummizubereitungen bevorzugt einsetzbar sind.

Es ist bekannt, daß die Hydroxylgruppen auf den Cyclodextrinmolekülen derivatisierbar sind. Die Reaktivität der Hydroxylgruppen an verschiedenen Positionen sowie die Verfahren zur Derivatisierung sind in verschiedenen Artikeln beschrieben (Liptak, A., Fugedi P., Szurmai, Z., Imre, J., Nanasi, P., Szejtli, J.: Proc. 1. Intern. Symp. für Cyclodextrine, Reidel, Dordrecht (1982) 275, Croft, A., Bartsch, R.A.: Tetrahedron 39 (1983) 1417).

Cyclodextrinderivate mit einem hohen Molekulargewicht, die mehrere Cyclodextrinringe in einem Molekül enthalten, sind durch Vernetzung mit bi- oder polyfunktionalen Reagenzien (z.B. Diepoxiden, Diisocyanaten), die mit den Hydroxylgruppen reagieren können, herstellbar (Zsadon, B., Fenyvesi,E.: Proc. 1. Intern. Symp. für Cyclodextrine, Reidel, Dordrecht (1982) 327).

Diese sogenannten Cyclodextrinpolymere können von einem niedrigeren Molekulargewicht sein und sind dann wasserlöslich oder sie weisen ein höheres Molekulargewicht auf und sind dann in Wasser quellfähig. Nicht alle Hydroxylgruppen sind mit Querverbindungen besetzt; die verbleibenden Hydroxylgruppen können mit anderen funktionellen Gruppen substituiert sein. Derart hergestellte Cyclodextrinpolymere sind für verschiedene Zwecke verwendbar.

Mit ionischen Gruppen modifizierte Cyclodextrinpolymere sind bekannt. Die wasserlöslichen Polymere mit ionischen Gruppen (Ung. Patent 191 101 (1986), US-Patent 4 545 152) werden bevorzugt in der Fotoindustrie verwendet (Szucs, M., Kiss, I.: Inf. Rec. mater, 14 (1986) 383 und 16 (1988) 439). Die Polymere mit den ionischen Gruppen, die im Wasser quellfähig sind, werden als spezifische Ionenaustauscher mit Komplexbildungsfähigkeit verwendet. Cyclodextrine mit Carboxymethyl- und Sulfalkylgruppen besitzen Kationenaustausch-, die mit Diallylaminogruppen substituierten Anionenaustauscheigenschaften (Jap. Patente 59 26142, 59 150549, 59 36549, 1984). Cyclodextrinpolymere mit Aminoalkylaminogruppen sind zur Enzymimmobilisierung verwendbar (Jap. Patent 60248729, 1986). N-Methyl-dihydronicotinamid-β-cyclodextrinpolymer ist als künstliches Enzym patentiert ( JP-62275102, 1987 ).

Es ist eine Aufgabe der Erfindung, neue Cyclodextrinpolymerderivate mit lipophilen Eigenschaften herzustellen.

Diese Aufgabe wird erfindinngsgemäß durch lipophile Cyclodextrinderivate der folgenden allgemeinen Formel gelöst:

$$\overset{R^{2\prime}{}_{s}}{\overset{|}{CD}}-\left\{-O-(-CH_2-\overset{R^2}{\overset{|}{CH}}-R-O-)_m—\overset{R^{2\prime}{}_{t}}{\overset{|}{CD}}-\left[-(-O-CH_2-\overset{R^2}{\overset{|}{CH}}-R'-)_n-X\right]_r\right\}_p$$

wobei

CD aus einem α-, β- und/oder gamma-Cyclodextrinmolekül durch Abspaltung von p+s- oder 1+t+r-Hydroxylgruppen stammt,

R und R'unabhängig voneinander aus der folgenden Gruppe ausgewählt werden :

$-CH_2-$ , $-CH0H-CH_2-$ , $-CH_2-0-(CH_2)_2-0-CH_2-CH0H-CH_2-$ , $-CH_2-0-CH_2-CHOH-CH_2-$ or $-CH_2-0-(CH_2)_4-0-CH_2-CHOH-CH_2-$

x $OR^1$ oder $R^2$ bedeutet, wobei

$R^1$ ein Wasserstoffatom oder - falls r nicht 0 ist - eine Gruppe darstellt, welche durch die Abspaltung eines Wasserstoffatoms aus einer Hydroxylgruppe aus einem $\alpha$-, $\beta$- oder $\gamma$-Cyclodextrinmolekül erhalten wird.

$R^2$ gleiche oder verschiedene Bedeutungen aufweist, nämlich $0R^3$ oder eine Alkylthio-, Alkylsulfonyl-, Alkylsulfinyl- oder Aminoalkylgruppe mit 1-20 Kohlenstoffatomen, bevorzugt mit 1 bis 6 C-Atomen, oder eine Gruppe darstellt, welche durch die Abspaltung eines Wasserstoffatoms aus einer Hydroxylgruppe aus einem $\alpha$-, $\beta$- oder $\gamma$-Cyclodextrinmolekül erhalten wird,

$R^{2'}$ der Bedeutung von $R^2$ entspricht, jedoch keine der Gruppen darstellt, die aus den Cyclodextrinmolekülen erhalten wurden, welche durch die Abspaltung eines Wasserstoffatoms aus einer Hydroxylgruppe aus einem $\alpha$-, $\beta$- oder $\gamma$-Cyclodextrinmolekül erhalten werden,

$R^3$ eine Alkyl-Gruppe mit 1 - 20 C-Atomen, bevorzugt mit 1 bis 6 C-Atomen, oder eine Aralkyl-oder Alkylarylgruppe darstellt,

wobei die Arylgruppe aus einer aromatischen oder heteroaromatischen Verbindung abstammt, oder $R_3$ eine $C_1$ - $C_7$-Acylgruppe oder eine unsubstituierte oder $C_1$ - $C_5$-mono-, di- oder trisubstituierte Silylgruppe darstellt.

m und n unabhängig voneinander ein ganzzahliges von 1 - 10 sind,

r ein ganzzahliges von 0 - 23 ist,

p ein ganzzahliges von 0 - 24 ist,

s und t unabhängig voneinander ein ganzzahliges von 0 - 7 sind,

m, n, r und t, einschließlich in den Seitenketten, innerhalb einer Einheit je unterschiedlich sein können,

und, falls CD eine Gruppe ist, die aus einem $\alpha$-Cyclodextrin stammt, $p+s<18$ und $r+t<17$ sind,

und, falls CD eine Gruppe ist, die aus einem $\beta$-Cyclodextrin stammt, $p+s<21$ und $r+t<20$ sind,

und, falls CD eine Gruppe ist, die aus einem gamma-Cyclodextrin stammt, $p+s<24$ und $r+t<23$ sind.

Die Alkylgruppen umfassen sowohl n-Alkylgruppen als auch ihre Isomere, bevorzugt $C_1$ - $C_{20}$-Alkylgruppen, insbesondere bevorzingt $C_1$ - $C_{10}$-Alkylgruppen.

Die Arylgruppen umfassen insbesondere Phenyl-, Naphthyl-, Biphenyl-, Anthracyl-Gruppen.

Die Alkylarylgruppen umfassen insbesondere Tolyl-, Xylyl-, Ethylphenyl-, Propylphenyl-, tertiäre Butylphenyl-Gruppen.

Die Aralkylgruppen umfassen insbesondere Benzyl-, Phenethyl-, Benzhydryl- und Trityl-Gruppen.

Die Heteroarylgruppen umfassen insbesondere Piridyl-, Picolyl-, Bipyridyl- und Collidyl-Gruppen.

Die Acylgruppen umfassen insbesondere Formyl-, Acetyl-, Propionyl-, Butyryl-, Isobutyryl-, Valeryl-, Isovaleryl-, Stearyl-, Oleyl- und Palmitoyl-Gruppen.

Die S-enthaltenden Gruppen umfassen insbesondere Methylsulphonyl-, Ethylsulphonyl-, Hexylsulphonyl-, Dodecylsulphonyl-, Methylthio-, Butylthio-, Hexylthio-Gruppen.

Die N-enthaltenden Gruppen umfassen insbesondere Amino-, Methylamino-, Ethylamino-, Propylamino-, Hexylamino-, Dodecylamino- und Imidazoyl-Gruppen.

Die Silylgruppen umfassen insbesondere Trimethylsilyl- und tertiäre Butyldimethylsilyl-Gruppen.

Das Cyclodextrin ist bevorzugt ein $\beta$-Cyclodextrinmolekül.

Weiterhin bevorzugt sind die erfindungsgemäßen lipophile Cyclodextrinpolymere,

dadurch gekennzeichnet,

daß m und n unabhängig voneinander ein Ganzzahliges von 3 bis 7 sind und/oder r ein Ganzzahliges von 5 bis 17 oder von 8 bis 14 ist, und/oder b ein Ganzzahliges von 5 bis 17 oder von 8 bis 14 ist, und/oder s und t unabhängig voneinander ein Ganzzahliges von 2 bis 5 sind und/oder m, n, r und t, einschließlich in den Seitenketten, innerhalb einer Einheit je unterschiedlich sein können, und, falls CD eine Gruppe ist, die aus einem $\alpha$-Cyclodextrin stammt, $p+s<13$ und $r+t<14$ sind oder $p+s<10$ und $r+t<10$ sind, und, falls CD eine Gruppe ist, die aus einem $\beta$-Cyclodextrin stammt, $p+s<17$ und $r+t<16$ sind und/oder $p+s<10$ und $r+t<9$ und, falls CD eine Gruppe ist, die aus einem gamma-Cyclodextrin stammt, $p+s<20$ und $r+t<19$ sind und/oder $p+s<14$ und $r+t<13$ sind.

Diese lipophilen Cyclodextrinpolymere sind in Lösungsmitteln mit einer niedrigen Dielektrizitätskonstante löslich oder quellbar.

Die lipophilen Cyclodextrinpolymere sind erfindungsgemäß durch zwei Verfahren herstellbar :

a) Durch Vernetzung der geeigneten Cyclodextrinderivate oder

b) durch Derivatisierung der Cyclodextrinpolymere.

a) Geeignete Cyclodextrinderivate sind Alkylderivate, bevorzugt Dimethyl-, Hydroxypropyl- und/oder Palmityl-Derivate, Acylderivate, z.B. Acetyl-, Benzoyl-, Succinyl-, Lauryl- und/oder Palmitoylderviate,

Alkylthioderivate, z.B. Methylthioderivate, Alkylsulfonylderivate, z.B. Propylsulfonylderivate, Alkylsulfinylderivate, z.B. Ethylsulfinylderivate und Dodecylsulfinylderivate, Desoxyaminoalkylderivate, z.B. Aminoethylderivate, Silylderivate, z.b. Dimethylsilyl-, Trimethylsilyl- und Tert. Butyl-dimethylsilylderivate, wobei der Substitutionsgrad im Bereich von 0,1 - 2,5 , bevorzugt zwischen 0,5-2,0, liegt.

Der Substitutionsgrad (D.S.) bedeutet die durchschnittliche Zahl an Substituenten auf einer Glucoseeinheit.

Der Substituent auf dem Polymer kann, wenn das erfindungsgemäße Cyclodextrinpolymer durch Derivatisierung des bereits polymerisierten Cyclodextrins hergestellt wird, nicht nur auf den Glucoseeinheiten des Cyclodextrinrings lokalisiert sein, sondern auch auf den verbindenden Glycerylbrücken. Es ist deshalb besser, das Produkt durch die Menge an Substituent in einem Gramm Produkt ausgedrückt in Mol. (meq = mole equivalent) zu charakterisieren.

D.S. = 1 entspricht 3 mMol/g oder 3 meq/g.

Das Produkt mit der größten Substituentenmenge in dieser Patentanmeldung ist das Acetyl-BCD mit 4,2 mMol/g (D.S. = 1,4) Acetylgehalt.

Die erfindinngsgemäßen Verbindungen können durch bekannte Verfahren hergestellt werden. Durch die Umsetzung mit Epichlorhydrin, Diepoxiden oder anderen bifunktionellen Reagenzien können die Polymerderivate der lipophilen Ainsgangsverbindungen hergestellt werden.

b) Cyclodextrinpolymere zur Herstellung ihrer lipophilen Derivate sind erfindungsgemäß lösliche oder quellfähige Produkte, die durch Polykondensation aus $\alpha$-, $\beta$- und/oder gamma-Cyclodextrin hergestellt werden, bevorzingt durch Vernetzung mit Epichlorhydrin oder Diepoxiden. Diese Polymere können durch solche chemischen Reaktionen modifiziert werden, die zur Derivatisierung der Cyclodextrine selbst verwendet werden.

Die Reaktionsbedingungen müssen gemäß den Eigenschaften des Cyclodextrinpolymers ausgewählt werden, d.h. das Reaktionsmedium muß so gewählt werden, daß sich das Polymer entweder löst oder daß es quillt, wobei die Polymerstruktur nicht zerstört werden darf. Alkalische wäßrige Lösungen, N,N-Dimethylformamid, Dimethylsulfoxid und Pyridin sind hierbei bevorzugt.

Erfindungsgemäß werden die Cylcodextrinderivate bevorzugt vernetzt, um ein Polymer herzustellen:

1.a) Das Cyclodextrinderivat wird in einer alkalischen wäßrigen Lösung gelöst oder suspendiert. Die Cyclodextrinkonzentration in der Lösung ist im Bereich von 20 - 50 Gew.-%, bevorzugt 30 - 40 Gew.-%, wenn das gewünschte Produkt löslich ist und im Bereich von 40 - 50 Gew.-%, wenn es ein quellfähiges Polymer ist. Die Alkali- oder Erdalkalihydroxydkonzentration, bevorzugt NaOH oder KOH, ist 2 - 8 Gew.-%, wenn ein Diepoxid zur Vernetzung verwendet wird. Wenn Epichlorhydrin als Vernetzungsmittel verwendet wird, ist das molare Verhältnis von Alkalihydroxyd zu Epichlorhydrin 0,8 - 1,2 : 1, bevorzugt 1 : 1. Das Vernetzungsmittel (Epichlorhydrin, 1,2,9,10-Diepoxy-4,7-dioxadecan, 1,2,11,12-Diepoxy-4,9-dioxadodecan) wird tropfenweise unter Rühren bei 20 - 80 °C zugegeben. Das Molverhältnis des Vernetzungsmittels zum Cyclodextrinderivat beträgt 5 - 20 : 1, bevorzugt 10 - 15 : 1 . Die Reaktionsmischung wird für weitere 1 - 4 Stunden gerührt und anschließend abkühlen gelassen. Falls das Produkt ein lösliches Polymer ist, wird es durch Dialyse oder Chromatographie gereinigt und bevorzugt durch Lyophilisation getrocknet. Falls das Prodinkt ein quellfähiges Polymer ist, wird es mit Wasser bis zur Neutralität gewaschen, mit wäßrigem Aceton bei zunehmender Acetonkonzentration dehydriert und getrocknet.

1.b) In einer weiteren Ausführungsform der Erfindung wird das Cyclodextrinderivat in Dichlormethan, Dichlorethan, Pyridin, Dimethylformamid oder Dimethylsulfoxid, bevorzugt in Dichlormethan oder Dichlorethan, gelöst und als Vernetzungsmittel bevorzugt Epichlorhydrin oder 1,2,9,10-Diepoxy-4,7-dioxadecan zugegeben. Nach dem Rühren wird als Katalysator bevorzugt Trifluoressigsäureanhydrid oder Bortrifluoridetherat zugegeben. Die Cyclodextrinkonzentration in der Lösung beträgt 10 - 50 Gew.-%. Das Molverhältnis des Vernetzungsmittels zum Cyclodextrinderivat beträgt 50 - 20 : 1 , bevorzugt 10 - 15 : 1. Das Reaktionsgemisch wird für weitere 1 - 4 Stunden gerührt und anschließend abkühlen gelassen. Wenn das Produkt löslich ist, wird ein 1 - 5-facher Wasserüberschuß, bezogen auf das Volumen des Reaktionsgemisches, zugegeben und das organische Lösungsmittel abgedampft und die wäßrige Lösung dialysiert und lyophilisiert. Wenn das Produkt ein quellfähiges Polymer ist, wird es mit dem gerade verwendeten organischen Lösungsmittel und anschließend mit Ethanol und/oder Aceton gewaschen und anschließend getrocknet.

2. Herstellung von alkylierten Cyclodextrinpolymeren

Die alkylierten Cyclodextrinpolymere können durch Verbindung der alkylierten Cyclodextrine gemäß den in 1.a) und 1.b) beschriebenen Verfahren hergestellt werden oder durch Alkylierung der Cyclodextrinpolymere. Im letzteren Fall werden 5 - 50 g des Cyclodextrinpolymers gelöst oder in 100 $cm^3$ einer wäßrigen Lösung von 0,1 - 10 Mol/l Alkali- oder Erdalkalihydroxid oder in Pyridin, Dimethylsulfoxid, N,N-Dimethylformamid, enthaltend 0 - 2 Mol Alkalihydroxid pro 1 Mol Alkylierungsmittel, gequollen. Das Alkylierungsmittel (Haloalkane, Alkylsulfate, Alkylepoxide) wird zu dieser Reaktionsmischung gegeben. Das Molverhältnis beträgt 1 - 25 Mole Alkylierungsmittel auf 1 Mol Cyclodextrin im Polymer. Die Alkylierung wird bei Raumtemperatur oder bei einer niedrigeren oder höheren Temperatur ausgeführt. Nach 0,5 - 24 Stunden Reaktionszeit werden die quellfähigen Polymere filtriert, gewaschen und getrocknet, die löslichen Polymere durch Dialyse oder Chromatographie gereinigt und lyophilisiert.

3. Herstellung von acylierten Cyclodextrinpolymeren

Acylierte Cyclodextrinpolymere können durch Verbindung acylierter Cyclodextrine gemäß den in 1.a) und 1.b) beschriebenen Verfahren oder durch Acylierung von Cyclodextrinpolymeren hergestellt werden. Die Acylierung wird in wäßriger Lösung, in organischer Lösung oder in organischer Lösung mit einem minimalen Wassergehalt (Pyridin, Essigsäure, N,N-Dimethylformamid) unter Verwendung organischer oder anorganischer Basen oder Säuren, wasserfreier oder wasser-enthaltender Lewis-Säuren (Alinminiumchlorid, Eisenchlorid, Bortrifluoridetherat, Zinkchlorid) oder ohne Katalyse mit einem geeigneten Säureanhydrid, Acylchlorid, geeigneten Estern oder kurzkettigen Alkoholen oder Cyclodextrinestern, durchgeführt.

Das Produkt kann durch allgemein verwendete Verfahren gereinigt werden :
Die Cyclodextrinmonomere durch Kristallisation,
die Cyclodextrinpolymere durch Waschen oder Dialyse,
beide Produkte durch geeignete chromatographische Verfahren.

Die acylierten Cyclodextrine können während der Polymerisation deacetyliert werden. Der Deacetylationsgrad hängt u.a. vom Substitutionsgrad des Ausgangsmaterials und von den Eigenschaften der Substituenten ab.

4. Herstellung von Stickstoff- oder Schwefel-enthaltenden lipophilen Cyclodextrinpolymeren

Stickstoff- und/oder Schwefel-enthaltende Cyclodextrinpolymere können durch Polymerisation geeigneter Cyclodextrinderivate gemäß dem in 1.a) und 1.b) beschriebenen Verfahren hergestellt werden.

5. Silylierte Cyclodextrinpolymere

Silylierte Cyclodextrinpolymere können durch Verbindung von silylierten Cyclodextrinmonomeren gemäß den in 1.a) und 1.b) beschriebenen Verfahren hergestellt werden oder durch Silylierung von Cyclodextrinpolvmeren mit Silylierungsmitteln (Chlortrimethylsilan, 1,1,1,3,3,3-Hexamethyl-disilazan, Tertbutyldimethylsilyl-chlorid) in absoluten organischen Lösungsmitteln (Pyridin, Ether, N,N-Dimethyl-formamid) in Gegenwart oder Abwesenheit organischer oder anorganischer Basen.

Das Produkt kann durch folgende allgemein gebräuchliche Verfahren hergestellt werden :
Die Cyclodextrinmonomere durch Kristallisation,
die Cyclodextrinpolymere durch Waschen oder Dialyse,
beide Produkte durch geeignete chromatographische Verfahren.

Die erfindungsgemäß hergestellten Produkte sind durch ihren Cyclodextringehalt gekennzeichnet, durch ihren Gehalt an Substituenten und durch den geschätzten Substitutionsgrad, bezogen auf den Cyclodextringehalt des Polymeren.

Die Lipophilität der Polymere kann durch ihren Lipophilitätsfaktor gekennzeichnet werden : Das Verhältnis des Sedimentationsvolumens in einem apolaren Lösungsmittel (Benzol) und in einem polaren Lösungsmittel (Wasser). Das Sedimentationsvolumen hängt von der Morphologie der Polymerpartikeln ab, weshalb nur Polymere mit einer ähnlichen Morphologie vergleichbar sind. Der Lipophilitätsfaktor der Cyclodextrinpolymere und der lipophilen Cyclodextrinpolymere beträgt 0,2 - 0,5 bzw. 0,3 - 2,0 .

Die erfindungsgemäß bereitgestellten Produkte behalten ihre Fähigkeit, Einschlußkomplexe zu bilden, ergänzt durch ihre hydrophoben Wechselwirkungen. Diese Produkte können als Sorbentien verwendet werden, um bestimmte Materialen zu adsorbieren und zu konzentrieren. Ihre Sorptionskapazität ist wesentlich höher als diejenigen der nicht-modifizierten Cyclodextrine.

BEISPIEL 1

Wasserlösliches β-Cyclodextrinpolymer aus Dimethyl-β-cyclodextrin

5 g (3,8 mMol) Dimethyl-β-Cyclodextrin wurden in 23 $cm^3$ Dichlorethan bei Raumtemperatur gelöst und hierzu 2,5 $cm^3$ (2,8 g, 1,6 mMol) 1,2,9,10-Diepoxy-4,7-dioxa-decan unter stetigem Rühren zugegeben. 0,25 $cm^3$ (2,2 mMol) Bortrifluoridetherat wurden in 2 $cm^3$ Dichlorethan gelöst und tropfenweise zur Reaktionsmischung zugegeben. Anschließend wurden 0,26 $cm^3$ (2,2 mMol) Bortrifluoridetherat, gelöst in 15 $cm^3$ Dichlorethan, zugegeben. Nach Beendigung der Reaktion wurde die Mischung in 500 $cm^3$ Wasser emulgiert und das Dichlorethan abgedampft. Die niedermolekularen Verunreinigungen wurden durch Dialyse entfernt. Nach der Abdampfung und der Lyophilisation wurden 5 g weißes Pulver erhalten.

Der Cyclodextringehalt, bestimmt durch iodometrische Titration nach saurer Hydrolyse, betrug 58,1 %. Der Gehalt an Methoxygruppen, bestimmt durch iodometrische Titration, betrug 23,1 %, 2,1 Mol pro Glucoseeinheit. Dies stimmt überein mit der geschätzten Zahl an 2 Mol Methoxygruppen pro Mol Glucoseeinheit.

Das Molekulargewicht des Produktes wurde durch seine Eigenviskosität und die gelchromatographische Molekulargewichtsverteilung bestimmt. Die Eigenviskositätszahl betrug 5,7 $cm^3$/g (Wasser 25 °C).
Die Chromatographiebedingungen waren wie folgt :

| | |
|---|---|
| Säule : | 160 $cm^3$ Ultrogel ACA-34 in einer Pharmacia K 16/100-Säule; |
| Probe : | 300 mg Produkt, gelöst in 5 $cm^3$ destilliertem Wasser |
| Eluierungsmittel : | Destilliertes Wasser |
| Eluierinngsgeschwindigkeit : | 30 $cm^3$/min |

5 $cm^3$ Proben wurden in einem LKB Ultro Frac-Sammler gesammelt und die optische Aktivität bestimmt. Das auf dem Gelchromatogramm basierende durchschnittliche Molekulargernicht der Probe betrug Mw = 3700, die Polydispersität 1,7.

BEISPIEL 2

Quellfähiges Polymer aus Dimethyl-β-cyclodextrin

2 g (2,6 mMol) Dimethyl-β-cyclodextrin wurden bei Raumtemperatur in 7 $cm^3$ Dichlorethan gelöst und hierzu 1 $cm^3$ (16 mMol) 1,2,9,10-Diepoxy-4,7-dioxa-decan unter stetigem Rühren zugegeben. 0,1 $cm^3$ (1,1 mMol) Bortrifluoridetherat wurden in 1 $cm^3$ Dichlorethan gelöst und tropfenweise zugegeben. Nach 1 Stunde wurden 1 $cm^3$ (16 mMol) 1,2,9,10-Diepoxy-4,7-dioxa-decan und 0,1 $cm^3$ (1,1 mMol) Bortrifluoridetherat, gelöst in 2 $cm^3$ Dichlorethan, hinzugegeben. Das Gel-ähliche Produkt wurde mit Dichlorethan und Methanol gewaschen. Die Ausbeute an Produkt betrug 2,5 g .

Sedimentationsvolinmen : 2,0 $cm^3$ /g in Benzol, 2,9 $cm^3$ /g in Wasser.

Die Quellfähigkeit ist gekennzeichnet durch den Lipophilitätsfaktor, der bestimmt ist durch das Vehältnis des Sedimentationsvolumens in Benzol und in Wasser : $V_B/V_W$ = 0,8.

BEISPIEL 3

Lösliches Polymer aus acetyliertem Dimethyl-β-cyclodextrin

1,8 g (1,1mMol) Peracetyl-dimethyl-β-cyclodextrin und 0,2 g (0,1 mMol) Peracetyl-β-cyclodextrin wurden in 3 $cm^3$ Wasser, enthaltend 0,5 g (12 mMol) Natriumhydroxid, bei 60 °C gelöst. Hierzu wurde 1 $cm^3$ (12 mMol) Epichlorhydrin tropfenrneise zugegeben und anschließend 2,5 Stunden lang gerührt. Die Reaktionsmischung wurde anschließend abgekühlt. Die alkalische wäßrige Lösung wurde vom sedimentierten, honig-ähnlichen Produkt dekantiert. Das Produkt wurde in destilliertem Wasser gelöst, Natriumchlorid und Alkali durch Dialyse entfernt und lyophilisiert. Es wurde ein weißes Pulver erhalten, welches in Wasser, Aceton und Benzol löslich war.

| | |
|---|---|
| Cyclodextringehalt : | 64 % |
| Eigenviskositätszahl : | 5,3 $cm^3$ /g |
| Geschätztes durchschnittliches Molekulargewicht : | 3300. |

BEISPIEL 4

Methylieruno von β-Cyclodextrin-Perlpolymer

1 g β-Cyclodextrin-Perlpolymer (hergestellt gemäß ungarischem Patent 177.419, β-Cyclodextringehalt : 46 %, (0,4 mMol) wurden in 300 $cm^3$ 30 % (2,25 Mol) Natriumhydroxidlösung gequollen und hierzu 56,7 g (0,36 Mol) Dimethylsulfat tropfenweise bei 0 °C zugegeben. Die Reaktionsmischung wurde bei Raumtemperatur 3 Stunden lang gerührt. Das Prodinkt wurde abgefiltert, von Alkali freigewaschen und bei 105 °C 1 Stunde lang getrocknet.

| | |
|---|---|
| Methoxygruppengehalt : | 11,7 % |
| Sedimentationsvolumen : | 3,1 $cm^3$/g in Benzol, 4,0 $cm^3$/g in Wasser. |
| Sedimentationsvolumen des Ausgangspolymers : | 2,2 bzw. 4,6 $cm^3$/g. |

Der Lipophilitätsfaktor nahm von 0,5 auf 0,8 zu.

BEISPIEL 5

Methylierung eines β-Cyclodextrin-Blockpolymeren

3 g eines β-Cyclodextrin-Blockpolymeren (β-Cyclodextringehalt : 48,2 %, 1,28 mMol vernetzt mit 1,2,9,10-Diepoxy-4,7-dioxa-decan) wurden in 120 $cm^3$ einer 30 % (0,9 Mol) Natriumhydroxidlösung bei 5 °C gerührt. Hierzu wurden tropfenweise 42,3 $cm^3$ (0,27 Mol) gekühltes Methylsulfat zugegeben; anschließend wurde die Mischung 1/2 Stunde lang bei 60 °C gerührt. Das Produkt wurde abgefiltert, durch wiederholtes Waschen mit destilliertem Wasser neutralisiert, mit wäßrigen Acetonlösungen zunehmender Konzentration dehydriert und bei 105 °C getrocknet, wobei 3,2 g Prodinkt erhalten wurde.

| | |
|---|---|
| Methoxygruppengehalt : | 6,2 % |
| Sedimentationsvolumen : | 1,8 $cm^3$/g in Benzol, 3,5 $cm^3$/g in Wasser. |
| Sedimentationsvolumen des Ausgangspolymeren : | 1,1 und 3,3 $cm^3$/g. |

Der Lipophilitätsfaktor nahm von 0,3 auf 0,5 zu.

BEISPIEL 6

Methylierung eines β-Cyclodextrin-Blockpolymeren mit einem Gehalt an Polyvinylalkohol

5,5 g eines β-Cyclodextrin-Blockpolymeren (β-Cyclodextringehalt : 48 %, 2,35 mMol Polyvinylalkoholgehalt : 5 %) wurden in 220 $cm^3$ einer 30 % Natriumhydroxidlösung bei -5 °C gerührt und tropfenweise 20,5 $cm^3$ (0,13 Mol) Dimethylsulfat zugegeben. Die Reaktionsmischung wurde 1/2 Stunde lang bei 60 °C gerührt. Das Produkt wurde, wie im Beispiel 5 beschrieben, abgefiltert, gewaschen und dehydriert. 5 g Prodinkt wurden erhalten.

| | |
|---|---|
| Methoxygruppengehalt : | 15,5 %. |
| Sedimentationsvolumen : | 3,6 $cm^3$/g in Benzol, 4,9 $cm^3$/g in Wasser. |
| Sedimentationsvolumen des Ausgangspolymeren : | 1,9 und 4,5 $cm^3$/g. |

Der Lipophilitätsfaktor erhöhte sich von 0,4 auf 0,7.

BEISPIEL 7

Butylierung eines α-Cyclodextrin-Perlpolymeren

10 g eines α-Cyclodextrin-Perlpolymeren (α-Cyclodextringehalt: 57,2 %, 5,9 mMol, Teilchengröße 80 - 250 µm) wurden in 100 $cm^3$ Dimethylsulfoxid bei Raumtemperatur gerührt. 5 g Natriumhydroxidpulver (0,125 Mol) wurden gelöst und während 1 Stunde 5 $cm^3$ (0,044 Mol) 1-Brombutan tropfenweise zugegeben. Die Reaktionsmischung wurde über Nacht gerührt, anschließend abgefiltert, mit verdünnter Salzsäure und Wasser gewaschen, mit Aceton dehydriert und bei 80 °C unter Vakuum getrocknet. 11,2 g Produkt wurden erhalten.

| | |
|---|---|
| Sedimentationsvolumen : | 4,3 $cm^3$/g in Benzol, 5,0 $cm^3$/g in Wasser. |
| Sedimentationsvolumen des Ausgangspolymeren : | 1,3 und 6,0 $cm^3$/g. |

Der Lipophilitätsfaktor nahm von 0,3 auf 0,5 zu.

BEISPIEL 8

Hvdroxyoropylierung eines Gamma-Cyclodextrin-Perlpolymeren

1,2 g (0,03 Mol) Natriumhydroxid wurden in 50 $cm^3$ Wasser gelöst und 10 g Gamma-Cyclodextrin-Perlpolymer (Cyclodextringehalt : 51 %, 3,9 mMol, Teilchengröße 80 - 250 $\mu$m) wurden in dieser Lösung gequollen und hierzu 10 $cm^3$ Propylenoxid (0,148 Mol) zugegeben. Die Reaktionsmischung wurde über Nacht gerührt, anschließend abgefiltert, neutralisiert, dehydriert und getrocknet. 17,1 g Produkt wurden erhalten.

| | |
|---|---|
| Sedimentationsvolumen : | 2,5 $cm^3$/g in Benzol, 4,3 $cm^3$/g in Wasser. |
| Sedimentationsvolumen des Ausgangspolymeren : | 1,9 bzw. 4,8 $cm^3$/g. |

Der Lipophilitätsfaktor nahm von 0,4 auf 0,6 zu.

BEISPIEL 9

6-Hydroxyhexyl-$\beta$-Cyclodextrin-Perlpolymer

5,0 g (2,2 Mol) eines $\beta$-Cyclodextrin-Perlpolymeren ($\beta$-Cyclodextringehalt : 51 %, 2,2 mMol, Teilchengröße 100 - 300 $\mu$m) wurden bei 80 °C unter stetigem Rühren in 40 $cm^3$ Wasser, enthaltend 4,0 g ( 0,071 Mol) Kaliumhydroxid, gequollen. 6-Chlor-1-hexanol (4 $cm^3$, 0,035 Mol) wurden zugegeben und für 24 Stunden gerührt. Nach Ende der Reaktion wurde das Polymer abgefiltert, mit Salzsäure gesäuert, mit Wasser zur Neutralität gewaschen, mit Aceton dehydriert und bei 80 °C unter Vakuum getrocknet.

| | |
|---|---|
| Erhaltenes Trockenmaterial : | 8,0 g. |
| Hydroxyhexylgehalt : | 60 % ( bezogen auf die Gewichtszunahme ), d.h. 1,4 Hydroxyhexylgruppen pro Cyclodextrineinheiten; |
| Sedimentationsvolumen : | 4,0 $cm^3$/g in Wasser, 1,5 $cm^3$/g in Benzol. |

Der Lipophilitätsfaktor erhöhte sich von 0,4 auf 0,6.

BEISPIEL 10

Acetyliertes $\beta$-Cyclodextrin-Perlpolymer

Verfahren 1.

100 g eines trockenen $\beta$-Cyclodextrin-Perlpolymeren ($\beta$-Cyclodextringehalt : 53,5 %, 47 mMol) wurden in wasserfreiem Pyridin (836 $cm^3$) und in Acetanhydrid (580 $cm^3$, 5,24 Mol) suspendiert. Nach 15minütigem Rühren bei Raumtemperatur wurde die Reaktionsmischung 16 Stunden lang stehen gelassen. Anschließend wurde für weitere 16 Stunden stetig gerührt (einschl. eines Abkühlungszeitraums auf Raumtemperatur) worauf eine fünfstündige Erhitzungsphase bei 60 °C folgte.

Das Polymer wurde abfiltriert, mit Aceton und Wasser gewaschen, mit wasserhaltiger Salzsäure verdünnt und anschließend wieder mit Wasser und Aceton gewaschen.

| | |
|---|---|
| Trockenmaterialausbeute : | 145 g; |
| Acetylgehalt : | 25,5 %, d.h. 16,8 Acetylgruppen pro Cyclodextrineinheiten; |
| Sedimentationsvolinmen : | 2,0 $cm^3$/g in Wasser; |
| | 2,2 $cm^3$/g in Ethanol; |
| | 3,0 $cm^3$/g in Methanol; |
| | 3,0 $cm^3$/g in Benzol; |

Der Lipophilitätsfaktor erhöhte sich von 0,4 auf 1,5.

Verfahren 2.

10,0 g eines $\beta$-Cyclodextrin-Perlpolymeren ( $\beta$-Cyclodextringehalt : 14,9 mMol, Teilchengröße : 10 - 100 $\mu$m) wurden in Acetanhydrid (40 $cm^3$) bei 25 °C suspendiert und hierzu wasserfreies Eisen-(III)-chlorid (0,32 g, 2 mMol) zur Suspension zugegeben.

Die Reaktionsmischung wurde 24 Stunden lang bei Raumtemperatur (25 °C) gerührt. Das Polymer wurde abfiltriert, zweimal zum Neutralisieren mit Wasser (20 $cm^3$) und anschließend zweimal mit Aceton (40 $cm^3$) gewaschen. Das Produkt (11,2)g wurde 6 Stunden lang bei 60 °C über Kaliumhydroxid und anschließend 4 Stunden lang bei 105 °C unter Vakuum getrocknet.

| | |
|---|---|
| Trockenmaterialausbeute : | 9,6 g; |
| Acetylgehalt : | mind. 0,5 %; |
| Sedimentationsvolumen : | 2,5 $cm^3$/g in Wasser; |
| | 0,7 $cm^3$/g in Benzol; |

Der Lipophilitätsfaktor erhöhte sich von 0,2 auf 0,3.

BEISPIEL 11

Palmitoyliertes $\beta$-Cyclodextrin-Perlpolymer

10,0 g eines $\beta$-Cyclodextrin-Perlpolymeren (4,5 mMol, $\beta$-Cyclodextringehalt: 51%, Teilchengröße : 100 - 300 $\mu$m) wurden in abs. Pyridin (50 $cm^3$) 30 Min. lang gequollen; anschließend wurde Palmitoylchlorid (17,5 g, 0,064 Mol) zugegeben und über Nacht gerührt. Das Polymer wurde abfiltriert, mit Wasser (10*50 $cm^3$) und Aceton gewaschen und anschließend bei 80 °C unter Vakuum getrocknet.

| | |
|---|---|
| Trockenmaterialausbeute : | 16,3 g; |
| Sedimentationsvolumen : | 2,2 $cm^3$/g in Wasser; |
| | 3,1 $cm^3$/g in Benzol (die Werte für das Ausgangspolymer sind 1,9 $cm^3$/g bzw. 4,8 $cm^3$/g); |

Der Lipophilitätsfaktor erhöhte sich von 0,4 auf 1,4.

BEISPIEL 12

Dodecylsulfonyl-$\beta$-Cyclodextrin-Perlpolymer

10,0 g eines $\beta$-Cyclodextrin-Perlpolymeren ( $\beta$-Cyclo-dextringehalt: 46%, Teilchengröße : 90 - 300 $\mu$m) wurden in Pyridin (50 $cm^3$) 30 Min. lang gequollen; anschließend wurde Dodecylsulfonylchlorid (8,8 g, 0,03 Mol) zugegeben und über Nacht gerührt. Das Polymer wurde abfiltriert, mit Wasser (10*50 $cm^3$) gewaschen, mit Aceton dehydriert und unter Vakuum getrocknet.

| | |
|---|---|
| Trockenmaterialausbeute : | 17,1 g; |
| Schwefelgehalt : | 1,9 %; |
| Sedimentationsvolumen : | 3,1 $cm^3$/g in Wasser; |
| | 2,7 $cm^3$/g in Benzol |

Der Lipophilitätsfaktor erhöhte sich von 0,5 auf 0,9.

BEISPIEL 13

N-Octylamino-$\beta$-Cyclodextrin-Perlpolymer

10,0 g eines N-Octylamino-desoxy-$\beta$-cyclodextrins (DS: 1,1) wurden in 20 % einer wäßrigen Natriumhydroxidlösung (10 $cm^3$, 12,5 mMol) bei 60 °C gelöst. Anschließend wurde Epichlorhydrin (4 $cm^3$) zugegeben, 30 Min. lang bei 60 °C gerührt und anschließend in Paraffinöl (30 $cm^3$) unter stetigem Rühren für weitere 5 Stunden dispergiert.

Das Polymer wurde abfiltriert, zum Neutralisieren mit Wasser gewaschen und mit Aceton dehydriert und anschließend bei 80 °C unter Vakuum getrocknet.

| | |
|---|---|
| Trockenmaterialausbeute : | 10,4 g; |
| $\beta$-Cyclodextringehalt : | 41 % |
| Stickstoffgehalt : | 2,1 %; |
| Sedimentationsvolumen : | 3,0 $cm^3$/g in Wasser; |
| | 2,3 $cm^3$/g in Benzol |
| Lipophilitätsfaktor : | 0,8 |

BEISPIEL 14

S-Butylthio-desoxy-$\beta$-cyclodextrin-Perlpolymer

S-Butylthio-desoxy-$\beta$-cyclodextrin (10,0 g, 5,4 mMol, DS: 0,85) wurden bei 60 °C unter Rühren in 5 % einer wäßrigen Natriumhydroxidlösung (15 $cm^3$, 18,8 mMol) unter einer inerten Atmosphäre gelöst. 1,2,11,12-Diepoxy-4,9-dioxa-dodecan ( 15 $cm^3$, 30 mMol) wurden zugegeben. Nach 10 Stunden wurde die

Lösung in Paraffinöl (50 $cm^3$) dispergiert. Das Polymer wurde nach 5 Stunden filtriert, mit Salzsäure gesäuert, zum Neutralisieren mit Wasser gewaschen, mit Aceton dehydriert und bei 80 °C unter Vakuum getrocknet.

| | |
|---|---|
| Trockenmaterialausbeute : | 10,5 g; |
| Partikelgröße : | 10 - 400 µm; |
| Cyclodextringehalt : | 43 % |
| Schwefelgehalt : | 2,0 %; |
| Sedimentationsvolumen : | 3,1 $cm^3$/g in Wasser; |
| | 2,5 $cm^3$/g in Benzol |
| Lipophilitätsfaktor : | 0,8 |

BEISPIEL 15

Trimethylsilylierung eines β-Cyclodextrin-Perlpolymeren

Verfahren 1.

10,0 g eines β-Cyclodextrin-Perlpolymeren (β-Cyclodextringehalt : 56 %, 4,9 mMol β-Cyclodextrin, Teilchengröße : 10 - 100 µm) wurden in abs. Diethylether (100 $cm^3$) bei 25 °C suspendiert. Trimethylsilylchlorid (4,35 g, 0,040 Mol) wurden auf einmal zugegeben. Zu diesem Reaktionsgemisch wurde bei 25 °C abs. Triethylamin (4,45 g, 0,044 Mol) tropfenweise zugegeben, bis zum Kochen erhitzt, 2 Stunden lang unter Rückfluß und weitere 18 Stunden bei 25 °C gerührt.
Das Polymer wurde abfiltriert, zweimal mit eiskaltem Wasser (20 $cm^3$) und anschließend zweimal mit Aceton (40 $cm^3$) gewaschen.

Das Produkt (14,8 g) wurde über Kaliumhydroxid 6 Stunden lang bei 60 °C und anschließend 4 Stunden lang bei 105 °C unter Vakuum getrocknet.

| | |
|---|---|
| Trockenmaterialausbeute : | 10,13 g; |
| Siliciumgehalt : | mind. 0,5 % |
| Sedimentationsvolumen : | 4,0 $cm^3$/g in Wasser; |
| | 1,1 $cm^3$/g in Benzol |

Der Lipophilitätsfaktor erhöhte sich von 0,2 auf 0,3.

Verfahren 2.

10,0 g eines β-Cyclodextrin-Perlpolymeren (Ausgangsmaterial vergleiche Beispiel 15, Verfahren 1.) wurden in abs. Pyridin (50 $cm^3$) und 1,1,1,3,3,3-Hexamethyldisilazan (1,91g, 0,012 Mol) bei 25 °C suspendiert. Hierzu wurde Trimethylsilylchlorid (2,15 g, 0,020 Mol) tropfenweise bei 25 °C zugegeben. Die Reaktionsmischung wurden auf 60 °C erhitzt, 2 Stunden lang bei 60 °C und weitere 18 Stunden bei 25 °C gerührt.
Das Polymer wurde abfiltriert, zweimal mit eiskaltem Wasser (20 $cm^3$) und zweimal mit Aceton (40 $cm^3$) gewaschen .
Das Produkt (14,6 g) wurde über Kaliumhydroxid 6 Stunden lang bei 60 °C und weitere 4 Stunden bei 105 °C unter Vakuum getrocknet.

| | |
|---|---|
| Trockenmaterialausbeute : | 10,22 g; |
| Siliciumgehalt : | mind. 0,7 % |
| Sedimentationsvolumen : | 4,0 $cm^3$/g in Wasser; |
| | 1,7 $cm^3$/g in Benzol |

Der Lipophilitätsfaktor erhöhte sich von 0,2 auf 0,4.

Verfahren 3.

10,0 g eines β-Cyclodextrin-Perlpolymeren (Ausgangsmaterial vergleiche Beispiel 15, Verfahren 1.) wurden in abs. N,N-Dimethylformamid (50 $cm^3$) und 1,1,1,3,3,3-Hexamethyldisilazan (4,04 g, 0,025 Mol) bei 25 °C suspendiert. Die Reaktionsmischung wurde bis zum Kochen erhitzt, 2 Stunden lang unter Rückfluß und weitere 18 Stunden bei 25 °C gerührt.
Das Polymer wurde abfiltriert, zweimal mit eiskaltem Wasser (20 $cm^3$) und zweimal mit Aceton (40 $cm^3$) gewaschen .
Das Produkt (15,2 g) wurde über Kaliumhydroxid 6 Stunden lang bei 60 °C und anschließend 4 Stunden bei

105 °C unter Vakuum getrocknet.

Trockenmaterialausbeute : 10,45 g;
Siliciumgehalt : mind. 1,4 %
Sedimentationsvolumen : 3,7 $cm^3$/g in Wasser;
1,2 $cm^3$/g in Benzol

Der Lipophilitätsfaktor erhöhte sich von 0,2 auf 0,3.

BEISPIEL 16

tertButyl-dimethylsilylierung eines β-Cyclodextrin-Perlpolymeren

10,0 g eines β-Cyclodextrin-Perlpolymer (β-Cyclodextringehalt: 56 %, 4,9 mMol β-Cyclodextrin, Teilchengröße : 10 - 100 μm) wurden in abs. Pyridin (40 $cm^3$) bei 25 °C suspendiert. tertButyl-dimethylsilylchlorid (3,77 g, 0,025 Mol) wurden tropfenweise zu abs. N,N-Dimethylformamid (10 $cm^3$) zugegeben, auf 60 °C erhitzt, 2 Stunden bei 60 °C und weitere 18 Stunden bei 25 °C gerührt.
Das Polymer wurde abfiltriert, zweimal mit eiskaltem Wasser (20 $cm^3$) und zweimal mit Aceton (40 $cm^3$) gewaschen .
Das Produkt (12,4 g) wurde über Kaliumhydroxid 6 Stunden lang bei 60 °C und weitere 4 Stunden bei 105 °C unter Vakuum getrocknet.

Trockenmaterialausbeute : 9,71 g;
Siliciumgehalt : mind. 0,5 %
Sedimentationsvolumen : 3,8 $cm^3$/g in Wasser;
1,3 $cm^3$/g in Benzol

Der Lipophilitätsfaktor erhöhte sich von 0,2 auf 0,3.

BEISPIEL 17

Phenolsorption aus wäßriger Lösung

Aus einem in Wasser gequollenen, methylierten β-Cyclodextrinperlpolymeren (BEISPIEL 4, 2,5g) wurde eine Chromatographiesäule ($V_o$ = 10 $cm^3$) hergestellt. Durch diese Säule wurde eine 0,1 % Phenollösung und nach der Regeneration eine 1 % Phenollösung geschickt. Nach einer anschließenden Regeneration wurde eine 3 % Phenollösung durch die Säule geschickt. 10 $cm^3$-Fraktionen wurden gesammelt. Der Phenolgehalt wurde durch Spektrophotometrie bei 267 nm bestimmt. Dieses Verfahren wurde mit dem Ausgangs-β-Cyclodextrin-Perlpolymeren ($V_o$ = 11,7 $cm^3$) wiederholt. Das sorbierte Phenol/ β-Cyclodextrin-Molverhältnis, das sorbierte Phenol-/1g β-Cyclodextrin-Perlpolymer-Verhältnis und das Volumen der Phenol-freien Fraktionen (vermindert um $V_o$) sind in der nachfolgenden Tabelle aufgeführt.

| Phenol (Ph0H) | Ausgangs-βCD-Perlpolymer | | | Methyliertes-βCD Perlpolymer | | |
|---|---|---|---|---|---|---|
| Konzentr. (%) | PhOH/βCD Molverh. | PhOH/Perlpolymer (g/g) | PhOH frei Vol. ($cm^3$) | PhOH/βCD Molverh. | PhOH/Perlpolymer (g/g) | PhOH frei Vol. ($cm^3$) |
| 0,1 | 0,53 : 1 | 0,02 | 28 | 0,78 : 1 | 0,03 | 55-60 |
| 1 | 4,52 : 1 | 0,17 | 13 | 8 : 1 | 0,30 | 40 |
| 3 | 7,57 : 1 | 0,28 | 3 | 23 : 1 | 0,87 | 35 |

BEISPIEL 18

p-Cresol-Sorption aus wäßrigen Lösungen

Eine p-Cresollösung (0,1%) wurde durch eine nach BEISPIEL 17 hergestellte Säule geschickt. Der p-Cresol-Gehalt wurde durch Spektrophotometrie bei 266 nm gemessen.
Im Fall des β-Cyclodextrin-Perlpolymeren :
$V_o$ = 11,7 $cm^3$;
Sorbiertes p-Cresol/-β-Cyclodextrin-Molverhältnis : 0,61 : 1;
p-Cresol-freies-Volumen (vermindert um $V_o$): 43 $cm^3$.

Im Fall des methylierten-β-Cyclodextrin-Perlpolymeren :
$V_o$ = 10 $cm^3$ ;
Sorbiertes p-Cresol/β-Cyclodextrin-Molverhältnis : 1,1 : 1;
p-Cresol-freies-Volumen (vermindert um $V_o$) : 105 $cm^3$ .

BEISPIEL 19

Benzol-Sorption aus wäßrigen Lösungen

Eine Benzollösung (0,04 %) wurde durch eine nach BEISPIEL 17 hergestellte Säule geschickt. Der Benzol-Gehalt wurde durch Spektrophotometrie bei 247 nm gemessen.
Im Fall des β-Cyclodextrin-Perlpolymeren :
$V_o$ = 11,7 $cm^3$;
Sorbiertes Benzol/β-Cyclodextrin-Molverhältnis : 0,54 : 1;
Benzol-freies-Volumen (vermindert um $V_o$): 38 $cm^3$.
Im Fall des methylierten-β-Cyclodextrin-Perlpolymeren :
$V_o$ = 10 $cm^3$ ;
Sorbiertes Benzol/β-Cyclodextrin-Molverhältnis : 0,63 : 1;
Benzol-freies-Volumen (vermindert um $V_o$) : 90 $cm^3$ .

Beispiel 20

2 g Polymere (A : β-Cyclodextrinpolymer (BCDP); B:Diethylenaminoethyl-BCDP (DEAE-BCDP); C: Carboxymethyl-BCDP (CM-BCDP)) wurden in 8 ml einer wäßrigen Ethanollösung (50 %v/v), mit einem Gehalt an 1,25 % Salicylsäure gequollen, zwei Stunden lang geschüttelt, bei Raumtemperatur über Nacht und anschließend bei 105°C weitere zwei Stunden lang getrocknet. Die Erzeugnisse sind gekennzeichnet durch ihren Gehalt an Salicylsäure und ihrer Auflösinngseigenschaften.

Der Salicylsäinregehalt wurde bestimmt durch wiederholtes Kochen der 0,25 g Probe in 20 ml Phosphatpufferlösung (pH = 7,2), Filtrierung und anschließender photometrischer Messung der Konzentration im abgekühlten Filtrat bei λ = 296 nm. Dieses Verfahren wurde vier Mal wiederholt und die Summe der Ergebnisse errechnet.

Die Auflösungsgeschwindigkeit wurde durch Rühren von 0,2 g der Probe mit einem Magnetrührer in 20 ml destilliertem Wasser oder in einer Phosphatpufferlösung von pH = 7,2 bei 37°C bestimmt. Proben aus dem Lösungsüberstand wurden zu bestimmten Zeiten (im Bereich von 1 - 60 Min) entnommen und die Konzentration spektrophotometrisch bestimmt. Die Erzeugnisse sind gekennzeichnet durch die gelöste Menge in 60 Min, ausgedrückt in Prozent der Gesamtaufnahme an aktivem Bestandteil.

| Beschreibung des β-CD-Polymers | | | Eigenschaften der Produkte | | |
|---|---|---|---|---|---|
| ionische Gruppe | Gehalt an ionischer Gruppe (meq/g) | Quellung (ml/g) | Salicylsäure Gehalt (%) | gelöste Menge in Wasser in 60 Min. (%) | gelöste Mengein Puffer in 60 Min. (%) |
| - | - | 5,0 | 4,3 | 54 | 81 |
| DEAE | 0,43 | 4,0 | 4,1 | 18 | 61 |
| CM | 0,62 | 3,6 | 4,4 | 58 | 99 |

Beispiel 21

1,2 g Ethacridinlactat werden in 90 ml 50% (v/v) Ethanol gelöst. 6 ml dieser Lösung werden zu 1,5 g β-Cyclodextrinpolymer zum Quellen gegeben. Die Suspension wird 1 Stunde lang geschüttelt, an der Luft zum Trocknen stehengelassen und anschließend bei 105° C zwei Stunden lang getrocknet.

Der Gehalt an Ethacridinlactat wird gem. Beispiel 1 bestimmt mit der Ausnahme, daß die Probe in einer Lösung aus etwa 0,1 g Benzoesäure in 50 % (v/v) Ethanol gekocht und anschließend spektrophotometrisch bei λ = 370 nm gemessen wird. Die Auflösungsgeschwindigkeit wird, wie im Beispiel 1 beschrieben, bestimmt.

| Beschreibung des β-CD-Polymers | | | Eigenschaften der Produkte | |
|---|---|---|---|---|
| ionische Gruppe | Gehalt an ionischer Gruppe (meq/g) | Quellung (ml/g) | Ethacridinlactat-Gehalt (%) | gelöste Menge in Wasser in 60 Min. (%) |
| - | - | 5,0 | 5,0 | 86 |
| CM | 0,04 | 5,0 | 5,2 | 70 |
| CM | 0,07 | 3,3 | 5,3 | 72 |
| CM | 0,09 | 3,7 | 4,9 | 61 |
| CM | 0,11 | 4,7 | 5,3 | 50 |
| CM | 0,17 | 4,3 | 5,2 | 27 |
| CM | 0,22 | 4,4 | 5,4 | 20 |
| CM | 0,35 | 4,8 | 5,4 | 7,7 |
| CM | 0,62 | 3,6 | 5,2 | 3,5 |
| CM | 0,73 | 3,1 | 4,7 | 1,5 |

Beispiel 22

0,24 g Methylenblau werden in 90 ml 50 % (v/v) Ethanol gelöst. 20 ml dieser Lösung werden zu 5 g β-Cyclodextrinpolymer zum Quellen gegeben. Die Suspension wird 2 Stunden lang geschüttelt, an der Luft zum Trocken stehengelassen und anschließend bei 105° C getrocknet.
Die Produkte werden wie im Beispiel 2 beschrieben bestimmt, wobei die spektrophotometrischen Messungen bei λ = 661 nm durchgeführt werden.

| Beschreibung des β-CD-Polymers | | | Eigenschaften der Produkte | |
|---|---|---|---|---|
| ionische Gruppe | Gehalt an ionischer Gruppe (meq/g) | Quellung (ml/g) | Methylenblau Gehalt (%) | gelöste Menge in Wasser in 60 Min. (%) |
| - | - | 5,0 | 1,2 | 34 |
| CM | 0,06 | 4,8 | 1,3 | 32 |
| CM | 0,62 | 3,6 | 1,2 | 9,2 |

**Patentansprüche**

1. Kaugummizubereitung mit erhöhter und zeitlich verlängerter Freisetzung der Inhalts- und/oder Wirkstoffe,
**dadurch gekennzeichnet,**
daß die Inhalts- und/oder Wirkstoffe mit einem quellfähigen Kohlenhydratpolymer einen Einschlußkomplex bilden.

2. Kaugummizubereitung nach Anspruch 1,
dadurch gekennzeichnet,
daß das quellfähige Kohlenhydratpolymer eine Stärke und/oder ein Stärkederivat ist, wobei das Stärkederivat insbesondere durch chemische und/oder enzymatische Reaktionen gewonnen wird.

**3.** Kaugummizubereitung nach einem oder mehreren der vorhergehenden Ansprüche, dadurch gekennzeichnet, daß das quellfähige Kohlenhydratpolymer ein Cyclodextrinpolymer und/oder ein Cyclodextrinpolymerderivat ist, wobei das Cyclodextrinpolymer und/oder dessen Derivat insbesondere ein $\alpha$-, $\beta$- oder $\gamma$-Cyclodextrinpolymer oder dessen Derivat ist.

**4.** Kaugummizubereitung nach einem oder mehreren der vorhergehenden Ansprüche, dadurch gekennzeichnet, daß das Cyclodextrinpolymer vernetzt ist, wobei die Cyclodextrinpolymere insbesondere mit einem bi- oder polyfunktionellen Reagens vernetzt sind.

**5.** Kaugummizubereitung nach einem oder mehreren der vorhergehenden Ansprüche, dadurch gekennzeichnet, daß das Cyclodextrin die folgende Formel aufweist :

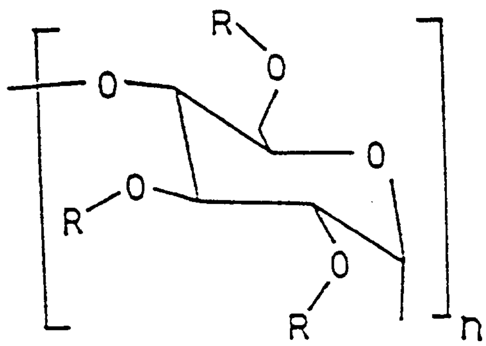

wobei n = 6 ($\alpha$-Cyclodextrin), 7 ($\beta$-Cyclodextrin) oder 8 ($\gamma$-Cyclodextrin) und R = H und/oder eine ionische Gruppe und/oder eine hydrophobe Gruppe und/oder eine vom Vernetzungsmittel stammende Gruppe ist, die zwei Cyclodextrinringe verbindet, und/oder eine vom Vernetzungsmittel stammende und mit einem Cyclodextrin monofunktionell umgesetzte Gruppe ist, und es in polymerer Form vorliegt.

**6.** Kaugummizubereitung nach einem oder mehreren der vorhergehenden Ansprüche, dadurch gekennzeichnet, daß die ionische Gruppe eine Carboxyalkyl-, Sulphalkyl-, Alkylamino- und/oder Dialkylaminogruppe ist, wobei die Alkylgruppe bevorzugt ein $c_1$ - $C_5$-Alkylrest ist.

**7.** Kaugummizubereitung nach einem oder mehreren der vorhergehenden Ansprüche, dadurch gekennzeichnet, daß die hydrophobe Gruppe eine Alkyl-, Arylalkyl-, Acyl-, Alkylsulphonyl-, Desoxyaminoalkyl- und/oder Silylgruppe ist, wobei der Alkylrest bevorzugt ein $C_1$ - $C_5$-Alkylrest ist.

**8.** Kaugummizubereitung nach einem oder mehreren der vorhergehenden Ansprüche, dadurch gekennzeichnet, daß der Alkylrest ein verzweigter und/oder unverzweigter $C_1$ - $C_5$-Alkylrest, bevorzugt ein $C_1$ - $C_3$-Alkylrest, ist, insbesondere, daß der Alkylrest ein Methyl-, Ethyl-, Propyl- oder Isopropylrest ist.

**9.** Kaugummizubereitung nach einem oder mehreren der vorhergehenden Ansprüche, dadurch gekennzeichnet, daß die vom Vernetzungsmittel stammende Gruppe, die zwei Cyclodextrinringe verbindet, eine Glyceryl-, Ethylenglykol-diglyceryl- und/oder Butylenglykoldiglyceryl-Gruppe ist.

**10.** Kaugummizubereitung nach einem oder mehreren der vorhergehenden Ansprüche, dadurch gekennzeichnet, daß die vom Vernetzungsmittel stammende und mit einem Cyclodextrin monofunktionell inmgesetzte

Gruppe eine Dihydroxypropyl-, 2-Hydroxy-3-[2-(2,3-dihydroxy)propoxy]ethoxy)propyl-, (2-Hydroxy-3-[2-(2,3-dihydroxy)propoxy]butoxy)propylgruppe ist.

**11.** Kaugummizubereitung nach einem oder mehreren der vorhergehenden Ansprüche, dadurch gekennzeichnet, daß das polymere Cyclodextrin 30 - 70 %, bevorzugt 50 - 60 % Cyclodextrin enthält.

**12.** Kaugummizubereitung nach einem oder mehreren der vorhergehenden Ansprüche, dadurch gekennzeichnet, daß das Cyclodextrin ein polymeres, dihydroxyalkyliertes Cyclodextrinderivat ist, wobei insbesondere das dihydroxyalkylierte Cyclodextrinderivat durch ionische Gruppen substituiert ist.

**13.** Kaugummizubereitung nach einem oder mehreren der vorhergehenden Ansprüche, dadurch gekennzeichnet, daß der Substitutionsgrad am Cyclodextrinring im Bereich von 0 - 21 liegt, wobei insbesondere der Substitutionsgrad bei Dimethyl-Cyclodextrin 14 ist und insbesondere der Substitutionsgrad bei Hydroxypropylcyclodextrin im Bereich von 1,2 - 8 liegt.

**14.** Kaugummizubereitung nach einem oder mehreren der vorhergehenden Ansprüche, dadurch gekennzeichnet, daß in Mischethern der Substitutionsgrad der Alkylsubstituenten bevorzugt im Bereich von 0 - 14 liegt, wobei die Summe der Substituenten im Bereich von 0 - 21 liegt.

**15.** Kaugummzubereitung nach einem oder mehreren der vorhergehenden Ansprüche, dadurch gekennzeichnet, daß als Kohlenhydratpolymer ein lipophiles Cyclodextrinpolymerderivat der folgenden allgemeinen Formel verwendet wird:

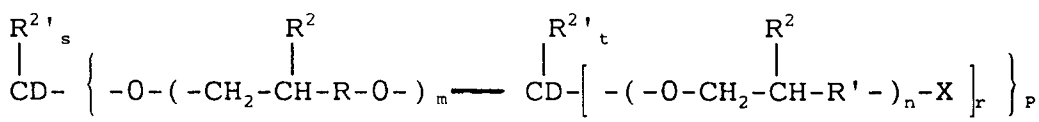

wobei
CD aus einem α-, β- und/oder γ-Cyclodextrinmolekül durch Abspaltung von p+s- oder 1+t+r-Hydroxylgruppen stammt,
R und R'unabhängig voneinander aus der folgenden Gruppe ausgewählt werden :
$-CH_2-$ , $-CHOH-CH_2-$ , $-CH_2-0-(CH_2)_2-0-CH_2-CH0H-CH_2-$ , $-CH_2-0-CH_2-CHOH-CH_2-$ or $-CH_2-0-(CH_2)_4-0-CH_2-CHOH-CH_2-$
X $OR^1$ oder $R^2$ bedeutet, wobei
$R^1$ ein Wasserstoffatom oder - falls r nicht 0 ist - eine Gruppe darstellt, welche durch die Abspaltung eines Wasserstoffatoms aus einer Hydroxylgruppe aus einem α-, β-oder γ-Cyclodextrinmolekül erhalten wird.
$R^2$ gleiche oder verschiedene Bedeutungen aufweist, nämlich $0R^3$ oder eine Alkylthio-, Alkylsulfonyl-, Alkylsulfinyl- oder Aminoalkylgruppe mit 1-20 Kohlenstoffatomen, bevorzugt mit 1 bis 6 C-Atomen, oder eine Gruppe darstellt, welche durch die Abspaltung eines Wasserstoffatoms aus einer Hydroxylgruppe aus einem α-, β- oder γ-Cyclodextrinmolekül erhalten werden,
$R^{2'}$ der Bedeutung von $R^2$ entspricht, jedoch keine der Gruppen darstellt, die aus den Cyclodextrinmolekülen erhalten wurden, welche durch die Abspaltung eines Wasserstoffatoms aus einer Hydroxylgruppe aus einem α-, β- oder γ-Cyclodextrinmolekül erhalten wird,
$R^3$ eine Alkyl-Gruppe mit 1 - 20 C-Atomen, bevorzugt mit 1 bis 6 C-Atomen, oder eine Aralkyl-oder Alkylarylgruppe darstellt, wobei die Arylgruppe aus einer aromatischen oder heteroaromatischen Verbindung abstammt, oder $R_3$ eine $C_1$ - $C_7$-Acylgruppe oder eine unsubstituierte oder $C_1$ - $C_5$-mono-, di- oder trisubstituierte Silylgruppe darstellt.
m und n unabhängig voneinander ein ganzzahliges von 1 - 10 sind,
r ein ganzzahliges von 0 - 23 ist,
p ein ganzzahliges von 0 - 24 ist,

s und t unabhängig voneinander ein ganzzahliges von 0 - 7 sind,
m, n, r und t, einschließlich in den Seitenketten, innerhalb einer Einheit je unterschiedlich sein können,
und, falls CD eine Gruppe ist, die aus einem $\alpha$-Cyclodextrin stammt, $p+s<18$ und $r+t<17$ sind,
und, falls CD eine Gruppe ist, die aus einem $\beta$-Cyclodextrin stammt, $p+s<21$ und $r+t<20$ sind,
und, falls CD eine Gruppe ist, die aus einem gamma-Cyclodextrin stammt, $p+s<24$ und $r+t<23$ sind.

**16.** Kaugummizubereitung nach einem oder mehreren der vorhergehenden Ansprüche,
dadurch gekennzeichnet,
daß die Kaugummigrundmasse eine wasser-unlösliche, natürliche oder synthetische Gummigrundmasse ist und
daß die Kaugummigrundmasse vorzugsweise einen Anteil von 5 - 50 Gew.-%, bevorzugt 15 - 30 Gew.-% der Kaugummizinsammensetzung aufweist.

**17.** Kaugummizubereitung nach einem oder mehreren der vorhergehenden Ansprüche,
dadurch gekennzeichnet,
daß sie Emulgatoren und/oder geschmacksbildende Zusätze und/oder Farbstoffe und/oder Süßstoffe enthält, wobei vorzugsweise die Emulgatoren Glycerin, Stearate, Oleate, Palmitate, Glyceride, Polyglycerolester, tierische, pflanzliche oder synthetische Wachse oder deren Mischungen sind und
als Stearate insbesondere Glycerylstearate und/oder Succrosestearate verwendet werden und
als Farbstoffe insbesondere Titandioxid, Indigoid und/oder Triphenylmethan-Farbstoffe verwendet werden.

**18.** Kaugummizubereitung nach einem oder mehreren der vorhergehenden Ansprüche,
dadurch gekennzeichnet,
daß die Wirkstoffe Analgetika, Antiarrhytmika, Antimykotika, antiinflammatorisch wirkende Mittel, Antipyretika, Antiinfektiva, Antihistaminika, psychotrop wirkende Mittel, Stimulantia, Sedativa, Tranquilizer, Laxativa, gefäßerweiternde- und gefäßverengende Mittel, Diuretika, Antiasthmatika, Expectorantia, Mucolytika, Antibiotika, Antitumormittel, Antitussiva, Antihypertonika, Antihypotonika, Antithrombotika, Antikariesmittel, Antiallergika, Lokalanästhetika, Vitamine, Aminosäuren und deren Derivate sowie deren Mischungen sind.

**19.** Kaugummizubereitung nach einem oder mehreren der vorhergehenden Ansprüche,
dadurch gekennzeichnet,
daß die Inhaltswirkstoffe natürliche und/oder künstliche Geschmacks- und/oder Süßstoffe sind, wobei
die Geschmacksstoffe vorzugsweise Grüne Minze, Zimt und/oder Pfefferminze sind und wobei
die Süßstoffe vorzugsweise Saccharose, Glucose, Dextrose, Sorbitol, Xylitol, Mannitol, Saccharin, Cyclamat und/oder Aspartam sind.

**20.** Kaugummizubereitung nach einem oder mehreren der vorhergehenden Ansprüche,
dadurch gekennzeichnet,
daß die natürlichen Süßstoffe in einer Menge von 20 - 60 Gew.-%, bevorzugt 40 - 60 Gew.-%, in der Kaugummizubereitung enthalten sind und
die künstlichen Süßstoffe in einer Menge von 0,001 -5 Gew.-%, bevorzugt etwa 0,05 - 1 Gew.-%, in der Kaugummizubereitung enthalten sind.

**21.** Kaugummizubereitung nach einem oder mehreren der vorhergehenden Ansprüche,
dadurch gekennzeichnet,
daß der oder die Wirkstoffe in Mengen von 0,001 - 1 Gew.-%, bevorzugt in einer Menge von 0,001 - 0,4 Gew.-%, in der Kaugummizubereitung enthalten sind.

**22.** Kaugummizubereitung nach einem oder mehreren der vorhergehenden Ansprüche,
dadurch gekennzeichnet,
daß das Kohlenhydratpolymer in Mengen von 1- 50 Gew.-%, bevorzugt in einer Menge von 2 - 10 Gew.-%, in der Kaugummizubereitung enthalten ist.

**23.** Kaugummizubereitung nach einem oder mehreren der vorhergehenden Ansprüche,
dadurch gekennzeichnet,
daß das molare Verhältnis von Wirkstoff : Cyclodextrin im Bereich von 1:5 - 3:1 liegt, wobei

das molare Verhältnis von Wirkstoff : Cyclodextrin bevorzugt 1:1 ist.

**24.** Kaugummizubereitung nach einem oder mehreren der vorhergehenden Ansprüche, dadurch gekennzeichnet, daß das molare Verhältnis von Hydrocortison : Cyclodextrin bevorzugt 1:2,2 ist.

**25.** Kaugummizubereitung nach einem oder mehreren der vorhergehenden Ansprüche, dadurch gekennzeichnet, daß die Teilchengröße des Kohlenhydratpolymers weniger als 100 µm, bevorzugt weniger als 60 µm beträgt und daß die Teilchengröße des Kohlenhydratpolymers mehr als 10 µm beträgt.

**26.** Kaugummizubereitung nach einem oder mehreren der vorhergehenden Ansprüche, dadurch gekennzeichnet, daß der Kohlenhydratpolymer-Wirkstoffkomplex in einer Menge von 1 - 30 Gew.-%, bevorzugt in einer Menge von 2 - 10 Gew.-%, in der Kaugummizubereitung enthalten ist.

**27.** Verfahren zur Herstellung der Kaugummizubereitung nach einem oder mehreren der vorhergehenden Ansprüche,
**dadurch gekennzeichnet,**
daß
a)
α) das Kohlenhydratpolymer in einer geeigneten Lösung der Inhalts-und/oder Wirkstoffe gequollen wird oder
β) das Kohlenhydratpolymer mit dem Inhalts-und/oder Wirkstoffen vermischt und anschließend in einer geeigneten Lösung gequollen wird,
b) diese Suspension nach Beendigung der Auflösung der Inhalts- und/oder Wirkstoffe und der Quellung getrocknet wird;
c) die Kaugummigrundlage unter Kneten erhitzt wird, anschließend
d) die Hilfsstoffe (Weichmacher, Emulgatoren, Geschmacks- und Farbmittel) zugegeben werden, anschließend
e) die Inhalts- und/oder Wirkstoff-Kohlenhydratpolymerzubereitung des Verfahrensschrittes b) zusammen mit dem Süßmittel oder getrennt hiervon zugegeben werden,
f) diese Kaugummizubereitungsmischung bis zur Erreichung einer homogenen Masse geknetet wird und anschließend
g) die Kaugummizubereitung in eine geeignete Form gebracht wird.

**28.** Verfahren nach einem oder mehreren der vorhergehenden Ansprüche, dadurch gekennzeichnet, daß zur Auflösung der Inhalts- und/oder Wirkstoffe und zum Quellen eine wäßrige oder eine wäßrig-alkoholische Lösung oder eine Pufferlösung verwendet wird.

**29.** Verfahren nach einem oder mehreren der vorhergehenden Ansprüche, dadurch gekennzeichnet, daß die Suspension nach Beendigung der Auflösung der Inhalts- und/oder Wirkstoffe und der Quellung - je nach den Eigenschaften der verwendeten Inhalts- und/oder Wirkstoffe - bei Zimmertemperatur oder bei erhöhten Temperaturen getrocknet wird.

**30.** Verfahren nach einem oder mehreren der vorhergehenden Ansprüche, dadurch gekennzeichnet, daß die Auflösung der Inhalts- und/oder Wirkstoffe und die Quellung bei Temperaturen von 0 - 60 °C, bevorzugt bei Zimmertemperatur, für 0,5 - 24 Stunden, bevorzugt für 1 - 6 Stunden, durchgeführt wird.

**31.** Verwendung der Kaugummizubereitung nach einem oder mehreren der vorhergehenden Ansprüche in Form einer pharmazeutischen Zubereitung.